# EUROPÄISCHE PATENTSCHRIFT

(11) **EP 1 282 824 B1**
(45) Veröffentlichungstag und Bekanntmachung des Hinweises auf die Patenterteilung: **15.03.2006**
(21) Anmeldenummer: 01931636.3
(22) Anmeldetag: 23.04.2001
(51) Int. Cl.: G01N 33/58, G01N 33/543

(54) **DOTIERTE NANOTEILCHEN ALS BIOLABEL**
DOPED NANOPARTICLES AS BIOLABELS
NANOPARTICULES DOPEES SERVANT DE MARQUEURS BIOLOGIQUES

(30) Priorität: 05.05.2000 DE 10021674; 12.02.2001 DE 10106643
(43) Veröffentlichungstag der Anmeldung: 12.02.2003
(73) Patentinhaber: Bayer Technology Services GmbH, 51368 Leverkusen (DE)
(72) Erfinder: HOHEISEL, Werner, 51061 Köln (DE); PETRY, Christoph, 47800 Krefeld (DE); HAASE, Markus, 22457 Hamburg (DE); RIWOTZKI, Karsten, 69117 Heidelberg (DE); BOHMANN, Kerstin, 50733 Köln (DE)
(86) Internationale Anmeldenummer: PCT/EP2001/004545
(87) Internationale Veröffentlichungsnummer: WO 2001/086299

(56) Entgegenhaltungen:
- WO-A-00/58731
- US-A- 5 593 783
- US-A- 5 893 999
- US-A- 5 990 479
- ZIJLMANS H J M A A ET AL: "Detection of cell and tissue surface antigens using up-converting phosphors: A new reporter technology." ANALYTICAL BIOCHEMISTRY, Bd. 267, Nr. 1, 1. Februar 1999 (1999-02-01), Seiten 30-36, XP002185942 ISSN: 0003-2697
- MEYSSAMY H ET AL: "WET-CHEMICAL SYNTHESIS OF DOPED COLLOIDAL NANOMATERIALS: PARTICLES AND FIBERS OF LAPO4:EU, LAPO4:CE, AND LAPO4:CE,TB" ADVANCED MATERIALS, VCH VERLAGSGESELLSCHAFT, WEINHEIM, DE, Bd. 11, Nr. 10, 9. Juli 1999 (1999-07-09), Seiten 840-844, XP000865123 ISSN: 0935-9648 in der Anmeldung erwähnt
- RIWOTZKI K ET AL: "Wet-chemical synthesis of doped colloidal nanoparticles: YVO4:Ln (Ln = Eu, Sm, Dy)" J PHYS CHEM B;JOURNAL OF PHYSICAL CHEMISTRY B DEC 10 1998 ACS, WASHINGTON, DC, USA, Bd. 102, Nr. 50, 10. Dezember 1998 (1998-12-10), Seiten 10129-10135, XP001034725 in der Anmeldung erwähnt
- ZOBEL H -P ET AL: "Cationic polyhexylcyanoacrylate nanoparticles as carriers for antisense oligonucleotides." ANTISENSE & NUCLEIC ACID DRUG DEVELOPMENT, Bd. 7, Nr. 5, Oktober 1997 (1997-10), Seiten 483-493, XP001041977 ISSN: 1087-2906

## Beschreibung

Die vorliegende Erfindung betrifft eine Nachweissonde für biologische Anwendungen, die lumineszierende anorganische dotierte Nanoteilchen (lad-Nanoteilchen) enthält.

Der Einsatz von Markern in biologischen Systemen zur Kennzeichnung oder Verfolgung spezifischer Substanzen ist seit Jahrzehnten ein etabliertes Instrument in der medizinischen Diagnostik und biotechnologischen Forschung. Solche Marker finden insbesondere Anwendung in der Flow Cytometrie, Histologie, in Immunoassays oder in der Fluoreszenz-Mikroskopie, bei letzterer zur Untersuchung biologischer und nicht-biologischer Materialien.

Die in der Biologie und Biochemie gängigsten Markersysteme sind radioaktive Isotope von Jod, Phosphor und anderen Elementen sowie Enzyme wie Meerrettich Peroxidase oder Alkalische Phosphatase, für deren Detektion spezielle Substrate benötigt werden. Außerdem werden als Marker zunehmend fluoreszierende organische Farbstoffmoleküle verwendet wie Fluorescein, Texas Red oder Cy5, die selektiv an eine bestimmte biologische oder andere organische Substanz angebunden sind. Je nach verwendetem System ist meistens ein weiteres Verbindungsmolekül oder eine Kombination von weiteren Verbindungsmolekülen oder Affinitätsmolekülen zwischen der nachzuweisenden Substanz und dem Marker notwendig, die die erforderliche spezifische Affinität zur eindeutigen Erkennung der nachzuweisenden Substanz aufweist. Die hierfür erforderliche Technik ist bekannt und z. B. beschrieben in "Bioconjugate Techniques", G.T. Hermanson, Academic Press, 1996 oder in "Fluorescent and Luminescent Probes for Biological Activity. A Practical Guide to Technology for Quantitative Real-Time Analysis", Second Edition, W.T. Mason, ed., Academic Press, 1999. Nach externer, meist elektromagnetischer Anregung des Markers wird dieser dann durch Emission von Fluoreszenzlicht die Präsenz der an den Marker angebundenen biologischen oder anderen organischen Substanzen anzeigen.

Nachteilig an den fluoreszierenden organischen Farbstoffmolekülen, die den heutigen Stand der Technik repräsentieren, ist, dass sie insbesondere bei Anwesenheit von Sauerstoff oder Radikalen zum Teil bereits nach einigen Millionen Lichtabsorptions-/Lichtemissionszyklen irreversibel geschädigt oder zerstört werden. Sie weisen also häufig eine für viele Anwendungen ungenügende Stabilität gegenüber einfallendem Licht auf. Weiterhin können die fluoreszierenden organischen Farbstoffmoleküle auch phototoxisch auf die biologische Umgebung wirken.

Ein weiterer Nachteil der fluoreszierenden, organischen Farbstoffe sind deren breite Emissionsbanden, die häufig einen zusätzlichen Ausläufer auf der langwelligen Seite des Fluoreszenzspektrums aufweisen. Dadurch wird ein "Multiplexing", das heißt die gleichzeitige Identifizierung mehrerer Substanzen, die mit jeweils unterschiedlichen Fluoreszenzfarbstoffen markiert sind, wegen der dann teilweise überlappenden Emissionsbanden behindert und die Anzahl der parallel nachweisbaren unterschiedlichen Substanzen stark begrenzt. Ein weiterer Nachteil bei der gleichzeitigen Verwendung mehrerer organischer Fluoreszenzfarbstoffe sind die relativ schmalen spektralen Anregungsbanden, innerhalb denen eine Anregung des Farbstoffs möglich ist. Um alle Farbstoffe effizient anregen zu können, müssen deshalb mehrere Lichtquellen, im allgemeinen Laser, oder ein komplizierter optischer Aufbau unter Verwendung einer Weißlichtquelle und einer geeigneten Anordnung von Farbfiltern verwendet werden.

Als alternative Marker zu den fluoreszierenden, organischen Farbstoffen wurden fluoreszierende anorganische Halbleiter-Nanokristalle vorgeschlagen. In der Patentschrift US 5,990,479 sowie in den PCT-Anmeldungen WO 00/17642 und WO 00/29617 wird offenbart, dass fluoreszierende anorganische Halbleiter-Nanokristalle, die der Klasse der II-VI- oder der III-V-Verbindungshalbleiter angehören und unter bestimmten Voraussetzungen auch aus Elementen der 4. Hauptgruppe des Periodensystems bestehen, als Fluoreszenzmarker in biologischen Systemen verwendet werden können. Durch Variation der Größe der Halbleiter-Nanokristalle kann durch Ausnutzung des so genannten "Quantum Size Effect" die Emissionswellenlänge des Fluoreszenzlichtes der Halbleiter-Nanokristalle im Spektralbereich des sichtbaren Lichts und des nahen Infrarot eingestellt werden. Die genaue Lage der Emissionswellenlänge hängt von der Festkörper-Bandlücke zwischen Leitungsband und Valenzband des gewählten Halbleitermaterials ab und wird durch die Teilchengröße bzw. durch deren Verteilung bestimmt. Halbleiter-Nanokristalle und ihre Verwendung als biologische Marker ist weiterhin offenbart in Warren C. W. Chan and Shuming Nie, Science, Vol. 281, 1998, Seiten 2016-2018 und Marcel Bruchez Jr., Mario Moronne, Peter Gin, Shimon Weiss, A. Paul Alivisatos, Science, Vol. 281, 1998, Seiten 2013-2016.

Die Halbleiter-Nanokristalle haben den Nachteil, dass sie mit höchster Präzision hergestellt werden müssen und somit nicht leicht zu produzieren sind. Da die Emissionswellenlänge des Fluoreszenzlichts von der Größe der Halbleiter-Nanokristalle abhängt, ist für eine schmale Bandbreite des Fluoreszenzlichts, das sich aus der Fluoreszenzlichtemission von einer Vielzahl einzelner Halbleiter-Nanokristallen zusammensetzt, erforderlich, dass die Größenverteilung der Halbleiter-Nanokristalle sehr eng ist. Um die für das Multiplexing erforderliche schmale Bandbreite des Fluoreszenzlichts zu gewährleisten, dürfen die Größenunterschiede zwischen den einzelnen Halbleiter-Nanokristallen nur wenige Angström, das heißt nur wenige Monolagen betragen. Dies stellt hohe Anforderungen an die Synthese der Halbleiter-Nanokristalle. Bei den Halbleiter-Nanokristallen wurden außerdem relativ schwache Quantenausbeuten bedingt durch strahlungslose Elektron-Loch-Paar Rekombinationen auf der Oberfläche der Halbleiter-Nanokristalle beobachtet. Aus diesem Grund wurde eine aufwändige Kern-Hüllen-Struktur vorgeschlagen, wobei der Kern aus dem eigentlichen Halbleitermaterial und die Hülle aus einem weiteren Halbleitermaterial mit einer größeren Bandlücke (z. B. CdS oder ZnS) besteht, das auf dem Kern so weit es möglich ist epitaktisch aufwächst. Um eine Anbindung dieser Kern-Hüllen-Teilchen an das nachzuweisende biologische Material zu erreichen, wurde zusätzlich noch eine weitere dünne Hülle aufgebracht, die vorzugsweise aus Silica-Glas (SiOₓ, x = 1-2) besteht (US 5,990,479, WO 99/21934, EP 1034234, Peng et al., Journal of the Arnerican Chemical Society, Vol. 119, 1997, Seiten 7019 - 7029). Eine solche multiple Kern-Hüllen-Struktur beinhaltet weitere relativ aufwändige Syntheseschritte. Nachteilig ist weiterhin, dass die Mehrzahl der aus der Literatur bekannten und fast alle bisher praktisch verwendeten Halbleiter-Nanokristalle Elemente enthalten, die als toxisch einzustufen sind wie z. B. Cadmium, Selen, Tellur, Indium, Arsen, Gallium oder Quecksilber.

Weiterhin können zum Nachweis spezieller biologischer Substanzen Kolloide aus Edelmetallen wie Gold oder Silber als Sonden eingesetzt werden. Die Oberflächen dieser Kolloide sind so modifiziert, dass eine Konjugation mit Biomolekülen möglich ist. Der Nachweis der Kolloide erfolgt über Messung der Lichtabsorption bzw. des elastisch gestreuten Lichts nach Einstrahlung von weißem Licht. Durch Anregung der Oberflächenplasmaresonanz der Metallteilchen, deren Wellenlänge spezifisch für das Material und für die Teilchengröße ist, kann so eine bestimmte Klasse von Teilchen und somit auch die entsprechenden Konjugate spezifisch erkannt werden (S. Schultz, D. R. Smith, J. J. Mock, D. A. Schultz; Proceedings of the National Academy of Science, Vol. 97, Issue 3, February 1, 2000, Seiten 996 - 1001). Durch den hohen Absorptions- und Streuquerschnitt ist der Nachweis sehr empfindlich. Nachteilig an dieser Lösung ist allerdings die relativ geringe Auswahl an verfügbaren Arbeitswellenlängen, so dass ein echtes Multiplexing nur eingeschränkt möglich ist. Außerdem ist die Effizienz, Licht zu streuen sehr stark vom Material und von der Teilchengröße abhängig, so dass die Nachweisempfindlichkeit eines nachzuweisenden Biomoleküls vom Material aber in hohem Maße von der Größe und somit von der Streufarbe des als Reporter wirkenden Metallteilchen abhängt.

In den Patentschriften US 4,637,988 und US 5,891,656 wird offenbart, dass Metall-Chelate mit einem Metallion aus der Reihe der Lanthaniden als Fluoreszenzmarker verwendet werden kann. Vorteilhaft an diesem System ist, dass die durch Lichtabsorption angeregten Zustände eine hohe Lebensdauer aufweisen, die bis in den Millisekundenbereich reichen. Dadurch kann der Nachweis der Reporterfluoreszenz zeitaufgelöst erfolgen, so dass Autofluoreszenzlicht nahezu vollständig unterdrückt werden kann. Allerdings haben diese Chelat-Systeme oft den Nachteil, dass deren Lumineszenz in wässrigen Medien, die für die meisten biologischen Anwendungen vorausgesetzt werden, in einem hohen Maße gelöscht wird. Deshalb müssen Chelate oft in einem zusätzlichen Schritt von der eigentlich nachzuweisenden Substanz separiert und in eine wasserfreie Umgebung gebracht werden (I.Hemmilä, Scand. J. Clin. Lab. Invest. 48, 1988, Seiten 389 - 400). Immunohistochemische Untersuchungen sind somit allerdings nicht möglich, da durch den Separationsschritt die Ortsinformation der Markierung verloren geht.

In den Patentschriften US 4,283,382 und US 4,259,313 wird offenbart, dass Polymer- (Latex-) Teilchen, in denen Metall-Chelate mit einem Metallion aus der Reihe der Lanthaniden eingebettet sind, ebenfalls als Fluoreszenzmarker verwendet werden können.

Leuchtphosphore, wie sie seit langer Zeit als Beschichtungsmaterial in Fluoreszenzlampen oder in Kathodenstrahlröhren verwendet werden, wurden ebenfalls als Reporterteilchen in biologischen Systemen verwendet. In US 5,043,265 wird offenbart, dass biologische Makromoleküle, die an Leuchtphosphorteilchen gekoppelt sind durch Messung der Fluoreszenz nachgewiesen werden können. Es wird ausgeführt, dass die Größe der Phosphorteilchen kleiner als 5 µm, bevorzugt kleiner als 1 µm sein sollte. Es wird allerdings auch erklärt, dass die Fluoreszenz der Teilchen mit kleiner werdendem Durchmesser rasch an Intensität verliert und die Teilchen deshalb größer als 20 nm und bevorzugt sogar größer als 100 nm sein sollten. Der Grund hierfür liegt offenbar u. a. in der Herstellungsmethode der Teilchen. Ausgehend von kommerziell erhältlichen Leuchtphosphoren mit einer Größe um 5 µm werden diese in einer Kugelmühle auf eine Größe von unter 1 µm herunter gemahlen. Nachteilig hieran ist, dass man durch diese Prozedur eine breite Größenverteilung der Teilchen und einen im allgemeinen relativ hohen Agglomerationsgrad erhält. Außerdem werden wahrscheinlich in die Kristallstruktur der Teilchen eine hohe Zahl von Defekten eingetragen, die die Quanteneffizienz der Fluoreszenzstrahlung erheblich verringern kann. Nachteilig ist weiterhin, dass sich die in der Erfindung offenbarten Teilchen mit einer Größe von meist mehreren 100 Nanometern und einer breiten Größenverteilung vielen Anwendungen, in der die Masse und Größe des Markers eine Rolle spielt, wie dies z. B. bei Anfärbungen von Zellbestandteilen oder beim Verfolgen von Substanzen der Fall ist, verschließen.

Spezielle Herstellungsverfahren von bestimmten Leuchtphosphoren mit Größen zwischen 1 nm und 100 nm, die auch für biologische Anwendungen nützlich sein sollen, werden in US 5,893,999 beansprucht. Darin wird ausgeführt, dass die Teilchen durch Gasphasensynthesen (Verdampfung und Kondensation, RF thermischer Plasma Prozess, Plasmaspritzen, Sputtern) und durch Hydrothermalsynthesen hergestellt werden können. Nachteilig an diesen Teilchen insbesondere für Anwendungen im Bereich der Biologie und Biochemie ist der hohe Agglomerationsgrad der Primärteilchen und somit zu die große Gesamtgröße der praktisch verwendbaren Agglomerate sowie die sehr breite Größenverteilung der verwendeten Teilchen, was die beschriebenen Herstellungsprozesse inherent mit sich bringen. Sowohl der Agglomerationsgrad als auch die breite Größenverteilung sind in zudem anhand der in der Patentschrift enthaltenen elektronenmikroskopischen Aufnahmen deutlich sichtbar.

Für den Einsatz als biologische Label werden spezielle Typen von Leuchtphosphoren in US 5,674,698 offenbart. Es handelt sich dabei um "Aufkonvertierende Phosphore" (upconverting phosphors), die die Eigenschaft haben, über einen Zwei-Photonen-Prozess Licht zu emittieren, das eine kleinere Wellenlänge besitzt als das absorbierte Licht. Durch Verwendung dieser Teilchen kann quasi hintergrundfrei gearbeitet werden, da solche Autofluoreszenz weitestgehend unterdrückt ist. Die Teilchen werden durch Mahlung und anschließender Temperung hergestellt. Die Teilchengröße liegt zwischen 10 nm und 3 µm, bevorzugt zwischen 300 nm und 1 µm. Nachteilig ist hier wieder die große Teilchengröße und die durch den Herstellungsprozess bedingten breiten Größenverteilung.

Eine Herstellungsmethode für diese "aufkonvertierenden" Leuchtphosphore, die ohne Mahlung auskommt, wird in US 5,891,361 und in US 6,039,894 offenbart. Es handelt sich hier um Fällungsprodukte, die durch nachträgliche teilweise reaktive Hochtemperaturbehandlungen in der Gasphase zu fluoreszierenden Phosphoren mit Größen zwischen 100 nm und 1 µm umgewandelt werden. Auch hier liegen die Nachteile wieder in den großen Teilchengrößen und in der breiten Größenverteilung, was mit den hohen Temperaturen bei der Synthese einhergeht.

In wissenschaftlichen Publikationen werden die Herstellung und Untersuchungen von Lumineszenzeigenschaften von ausgewählten lumineszierenden anorganischen dotierten Nanoteilchen behandelt. Die publizierten lumineszierenden anorganischen dotierten Nanoteilchen bestehen aus Oxiden, Sulfiden, Phosphaten oder Vanadaten, die mit Lanthaniden oder aber mit Mn, Al, Ag oder Cu dotiert sind. Diese lumineszierenden anorganischen dotierten Nanoteilchen fluoreszieren auf Grund ihrer Dotierung in einem engen Spektralbereich. Ein Anwendungspotential wird im Einsatz als Phosphore in Kathodenstrahlröhren oder als Lampenleuchtstoffe gesehen. Unter anderem ist die Herstellung der folgenden lumineszierenden anorganischen dotierten Nanoteilchen publiziert worden: YVO₄:Eu, YVO₄:Sm, YVO₄:Dy (K. Riwotzki, M. Haase; Journal of Physical Chemistry B; Vol. 102, 1998, Seiten 10129 bis 10135); LaPO₄:Eu, LaPO₄:Ce, LaPO₄:Ce, Tb; (H. Meyssamy, K. Riwotzki, A. Kornowski, S. Naused, M. Haase; Advanced Materials, Vol. 11, Issue 10, 1999, Seiten 840 bis 844); (K. Riwotzki, H. Meyssamy, A. Kornowski, M. Haase; Journal of Physical Chemistry B Vol. 104, 2000, Seiten 2824 bis 2828); ZnS:Tb, ZnS:TbF₃, ZnS:Eu, ZnS:EuF₃, (M. Ihara, T. Igarashi, T. Kusunoki, K. Ohno; Society for Information Display, Proceedings 1999, Session 49.3); Y₂O₃:Eu (Q. Li, L. Gao, D. S. Yan; Nanostructured Materials Vol. 8, 1999, Seiten 825 ff); Y₂SiO₅:Eu (M. Yin, W. Zhang, S. Xia, J. C. Krupa; Journal of Luminescence, Vol. 68, 1996, Seiten 335 ff.); SiO₂:Dy, SiO₂:Al, (Y. H. Li, C. M. Mo, L. D. Zhang, R. C. Liu, Y. S. Liu; Nanostructured Materials Vol. 11, Issue 3, 1999, Seiten 307 bis 310); Y₂O₃:Tb (Y. L. Soo, S. W. Huang, Z. H. Ming, Y. H. Kao, G. C. Smith, E. Goldburt, R. Hodel, B. Kulkarni, J. V. D. Veliadis, R. N. Bhargava; Journal of Applied Physics Vol. 83, Issue 10, 1998, Seiten 5404 bis 5409); CdS:Mn (R. N. Bhargava, D. Gallagher, X. Hong, A. Nurmikko; Physical Review Letters Vol. 72, 1994, Seiten 416 bis 419); ZnS:Tb (R. N. Bhargava, D. Gallagher, T. Welker; Journal of Luminescence, Vol. 60, 1994, Seiten 275 ff.).

Einen Überblick über die bekannten lumineszierenden anorganischen dotierten Materialien und ihre Verwendung als technische Phosphore, die eine Größe von einigen Mikrometern haben, gibt Ullmann's Encyclopedia of Industrial Chemistry, WILEY-VCH, 6^{th} edition, 1999, Electronic Release, Kapitel "Luminescent Materials: 1. Inorganic Phosphors". Die dort gegebene Übersicht bezieht sich ausschließlich auf die für die dort beschriebenen Anwendungen verwendbaren Materialklassen und nicht auf besondere Eigenschaften dieser Materialien in Form von Nanoteilchen.

US-A-5,893,999 offenbart lad-Nanoteilchen mit geringer Größe als Nachweissonden zu verwenden. Die beschriebenen Herstellmethoden führen aber nicht zu anwendbaren separierten Teilchen mit geringer Größe. Weiterhin sollen dieselben Teilchen als Nachweissonden einsetzbar sein. US-A-5,893,999 erwähnt dazu allein die Protein A-Goldkolloid-Methode. Aufgrund der Größe und des Agglomerationsgrades der Teilchen und dem einfachen Zusammengeben der Teilchen mit Protein A erscheint ein Einsatz als Nachweissonde allerdings zweifelhaft.

Meyssamy H et al. (Advanced materials, VCH Verlagsgesellschaft, Weinheim, DE, BD.11, Nr. 10, 9. Juli 1999 (1999-07.09), Seiten 840-844) und Ritwotzki K. et al. (j. Phys. Chem. B, Journal of physical chemistry B, DEC 10 1998 ACS, Washington DC, USA, BD. 102, Nr. 50, 10. Dezember 1998 (1988.12.10), Seiten 10129-10135) beschriebene Verfahren zur Bereitstellung von lad-Nanoteilchen führen zu kleinen Teilchen von 2-20 nm im mittleren Durchmesser. Gemäß der beschriebenen Synthesemethoden sind diese als Nachweissonden jedoch nicht direkt einsetzbar, weshalb in den genannten Schriften beispielsweise auch keinerlei Hinweis auf eine Verwendung in biologischen Systemen zu finden ist.

Die erfindungsgemäße Aufgabe besteht in der Bereitstellung einer Nachweissonde für biologische Anwendungen, die wenige Nanometer große, anorganische, lumineszierende Teilchen enthält.

Die Lösung der erfindungsgemäßen Aufgabe besteht in einer Nachweissonde für biologische Anwendungen enthaltend lumineszierende anorganische dotierte Nanoteilchen (lad-Nanoteilchen).

lad-Nanoteilchen sind mit Fremdionen derart dotiert, dass sie nach Anregung mit einer Strahlungsquelle durch Absorption und/oder Streuung und/oder Beugung dieser Strahlung oder durch Emission von Fluoreszenzlicht materialspezifisch nachgewiesen werden können. Die lad-Nanoteilchen können durch schmal- oder breitbandige elektromagnetische Strahlung oder durch einen Teilchenstrahl angeregt werden. Der qualitative und/oder quantitative Nachweis der Teilchen erfolgt durch Messung einer Änderung der Absorption und/oder Streuung und/oder Beugung dieser Strahlung oder durch Messung von materialspezifischem Fluoreszenzlicht bzw. dessen Änderung.

Die lad-Nanoteilchen haben eine nahezu sphärische Morphologie mit Ausdehnungen im Bereich von 1 nm bis 1 µm, bevorzugt im Bereich von 2 nm bis 100 nm, besonders bevorzugt im Bereich von 2 nm bis unter 20 nm und ganz besonders bevorzugt zwischen 2 nm und 10 nm. Unter Ausdehnungen wird der maximale Abstand von zwei auf der Oberfläche eines lad-Teilchens liegenden Punkte verstanden. Die lad-Nanoteilchen können auch eine ellipsoidförmige Morphologie besitzen oder facetiert sein mit Ausdehnungen, die in den oben angegeben Grenzen liegen. Die lad-Nanoteilchen können darüber hinaus auch eine ausgeprägte nadelförmige Morphologie aufweisen mit einer Breite von 3 nm bis 50 nm, bevorzugt von 3 nm bis unter 20 nm und einer Länge von 20 nm bis 5 µm, bevorzugt von 20 nm bis 500 nm. Die Teilchengröße kann mit der Methode der Ultrazentrifugation, der Gelpermeationschromatographie oder mittels Elektronenmikroskopie bestimmt werden.

Im Sinne der Erfindung geeignete Materialien für die lad-Nanoteilchen sind anorganische Nanokristalle, deren Kristallgitter (Wirtsmaterial) mit Fremdionen dotiert ist. Hierunter zählen insbesondere alle Materialien und Materialklassen, die als sogenannte Phosphore z. B. in Leuchtschirmen (z. B. für Elektronenstrahlröhren) oder als Beschichtungsmaterial in Fluoreszenzlampen (für Gasentladungslampen) Verwendung finden, wie sie zum Beispiel in Ullmann's Encyclopedia of Industrial Chemistry, WILEY-VCH, 6^{th} edition, 1999 Electronic Release, Kapitel "Luminescent Materials: 1. Inorganic Phosphors" genannt sind, sowie die aus dem oben zitierten Stand der Technik bekannten lumineszierenden anorganischen dotierten Nanoteilchen. In diesen Materialien dienen die Fremdionen als Aktivatoren für die Emission von Fluoreszenzlicht nach Anregung durch UV-, sichtbares oder IR-Licht, Röntgen- oder Gammastrahlen oder Elektronenstrahlen. Bei einigen Materialien werden auch mehrere Sorten von Fremdionen in das Wirtsgitter eingebaut, um einerseits Aktivatoren für die Emission zu erzeugen und um andererseits die Anregung des Teilchensystems effizienter zu gestalten oder um die Absorptionswellenlänge durch Verschiebung an die Wellenlänge einer gegebenen Anregungslichtquelle anzupassen (sogenannte Sensitizer). Der Einbau mehrerer Sorten von Fremdionen kann auch dazu dienen, gezielt eine bestimmte Kombination von Fluoreszenzbanden, die von einem Teilchen emittiert werden sollen, einzustellen.

Das Wirtsmaterial der lad-Nanoteilchen besteht vorzugsweise aus Verbindungen des Typs XY. Dabei ist X ein Kation aus Elementen der Hauptgruppen 1a, 2a, 3a, 4a der Nebengruppen 2b, 3b, 4b, 5b, 6b, 7b oder der Lanthaniden des Periodensystems. In einigen Fällen kann X auch eine Kombination bzw. Mischung aus den genannten Elementen sein. Y kann ein mehratomiges Anion, enthaltend ein oder mehrere Element(e) der Hauptgruppen 3a, 4a, 5a, der Nebengruppen 3b, 4b, 5b, 6b, 7b und/oder 8b sowie Elemente der Hauptgruppen 6a und/oder 7a, sein. Y kann aber auch ein einatomiges Anion aus der Hauptgruppe 5a, 6a oder 7a des Periodensystems sein. Das Wirtsmaterial der lad-Nanoteilchen kann auch aus einem Element der Hauptgruppe 4a des Periodensystems bestehen. Als Dotierung können Elemente der Hauptgruppen 1a, 2a oder aus der Gruppe enthaltend Al, Cr, Tl, Mn, Ag, Cu, As, Nb, Nd, Ni, Ti, In, Sb, Ga, Si, Pb, Bi, Zn, Co und/oder Elemente der Lanthaniden dienen. Auch Kombinationen von zwei oder mehreren dieser Elemente können in unterschiedlichen relativen Konzentrationen zueinander als Dotierungsmaterial dienen. Die Konzentration des Dotierungsmaterials im Wirtsgitter beträgt zwischen 10⁻⁵ mol% und 50 mol%, bevorzugt zwischen 0,01 mol% und 30 mol%, besonders bevorzugt zwischen 0,1 mol% und 20 mol%.

Bevorzugt werden Sulfide, Selenide, Sulfoselenide, Oxysulfide, Borate, Aluminate, Gallate, Silikate, Germanate, Phosphate, Halophosphate, Oxide, Arsenate, Vanadate, Niobate, Tantalate, Sulfate, Wolframate, Molybdate, Alkalihalogenide sowie andere Halogenide oder Nitride als Wirtsmaterialien für die lad-Nanoteilchen verwendet. Beispiele für diese Materialklassen sind zusammen mit den entsprechenden Dotierungen in der folgenden Liste angegeben (Materialien des Typs B:A mit B = Wirtsmaterial und A = Dotierungsmaterial):
LiI:Eu; NaI:Tl; CsI:T1; CsI:Na; LiF:Mg; LiF:Mg,Ti; LiF:Mg,Na; KMgF₃:Mn; Al₂O₃:Eu; BaFCI:Eu; BaFCI:Sm; BaFBr:Eu; BaFCl_{0,5}Br_{0,5}:Sm; BaY₂F₈:A (A = Pr, Tm, Er, Ce); BaSi₂O₅:Pb; BaMg₂Al₁₆O₂₇:Eu; BaMgAl₁₄O₂₃:Eu; BaMgAl₁₀O₁₇:Eu; BaMgAl₂O₃:Eu; Ba₂P₂O₇:Ti; (Ba,Zn,Mg)₃Si₂O₇:Pb; Ce(Mg,Ba)Al₁₁O₁₉; Ce_{0,65}Tb_{0,35}MgAl₁₁O₁₉:Ce,Tb; MgAl₁₁O₁₉:Ce,Tb; MgF₂:Mn; MgS:Eu; MgS:Ce; MgS:Sm; MgS:(Sm,Ce); (Mg,Ca)S:Eu; MgSiO₃:Mn; 3,5MgO·0,5MgF₂·GeO₂:Mn; MgWO₄:Sm; MgWO₄:Pb; 6MgO·As₂O₅:Mn; (Zn,Mg)F₂:Mn; (Zn₄Be)SO₄:Mn; Zn₂SiO₄:Mn; Zn₂SiO₄:Mn,As; ZnO:Zn; ZnO:Zn,Si,Ga; Zn₃(PO₄)₂:Mn; ZnS:A (A = Ag, Al, Cu); (Zn,Cd)S:A (A = Cu, Al, Ag, Ni); CdBO₄:Mn; CaF₂:Mn; CaF₂:Dy; CaS:A (A = Lanthanide, Bi); (Ca,Sr)S:Bi; CaWO₄:Pb; CaWO₄:Sm; CaSO₄:A (A = Mn, Lanthanide); 3Ca₃(PO₄)₂·Ca(F,Cl)₂:Sb,Mₙ; CaSiO₃:Mn,Pb; Ca₂AlSi₂O₇:Ce; (Ca,Mg)SiO₃:Ce; (Ca,Mg)SiO₃:Ti; 2SrO·6(B₂O₃)-SrF₂:Eu; 3Sr₃(PO₄)₂·CaCl₂:Eu; A₃(PO₄)₂·ACl₂:Eu (A = Sr, Ca, Ba); (Sr,Mg)₂P₂O₇:Eu; (Sr,Mg)₃(PO₄)₂:Sn; SrS:Ce; SrS:Sm,Ce; SrS:Sm; SrS:Eu; SrS:Eu,Sm; SrS:Cu,Ag; Sr₂P₂O₇:Sn; Sr₂P2O₇:Eu; Sr₄Al₁₄O₂₅:Eu; SrGa₂S₄:A (A = Lanthanide, Pb); SrGa₂S₄:Pb; Sr₃Gd₂Si₆O₁₈:Pb,Mn; YF₃:Yb,Er; YF₃:Ln (Ln = Lanthanide); YLiF₄:Ln (Ln = Lanthanide); Y₃Al₅O₁₂:Ln (Ln = Lanthanide); YAl₃(BO₄)₃:Nd,Yb; (Y,Ga)BO₃:Eu; (Y,Gd)BO₃:Eu; Y₂Al₃Ga₂O₁₂:Tb; Y₂SiO₅:Ln (Ln = Lanthanide); Y₂O₃:Ln (Ln = Lanthanide); Y₂O₂S:Ln (Ln = Lanthanide); YVO₄:A (A = Lanthanide, In); Y(P, V)O₄:Eu; YTaO₄:Nb; YAlO₃:A (A = Pr, Tm, Er, Ce); YOCI:Yb,Er; LnPO₄:Ce, Tb (Ln = Lanthanide oder Mischungen von Lanthaniden); LuVO₄:Eu; GdVO₄:Eu; Gd₂O₂S:Tb; GdMgB₅O₁₀:Ce, Th; LaOBr:Tb; La₂O₂S:Tb; LaF₃:Nd, Ce; BaYb₂F₈:Eu; NaYF₄:Yb,Er; NaGdF₄:Yb, Er; NaLaF₄:Yb, Er; LaF₃:Yb, Er, Tm; BaYF₅:Yb, Er; Ga₂O₃:Dy; GaN:A (A = Pr, Eu, Er, Tm); Bi₄Ge₃O₁₂; LiNbO₃:Nd, Yb; LiNbO₃:Er; LiCaAlF₆:Ce; LiSrAlF₆:Ce; LiLuF₄:A (A = Pr, Tm, Er, Ce); Li₂B₄O₇:Mn, SiOₓ:Er, Al (0 ≤ x ≤ 2).

Besonders bevorzugt werden folgende Materialien als lad-Nanoteilchen verwendet: YVO₄:Eu, YVO₄:Sm, YVO₄:Dy, LaPO₄:Eu, LaPO₄:Ce, LaPO₄:Ce,Tb, LaPO₄:Ce,Dy, LaPO₄:Ce,Nd, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, CdS:Mn, ZnS:Tb, ZnS:Ag, ZnS:Cu. Unter den besonders bevorzugten Materialien werden insbesondere diejenigen ausgewählt, die eine kubische Gitterstruktur des Wirtsgitters besitzen, da bei diesen Materialien die Zahl der einzelnen Fluoreszenzbanden minimal wird. Beispiele hierfür sind: MgF₂:Mn; ZnS:Mn, ZnS:Ag, ZnS:Cu, CaSiO₃:Ln, CaS:Ln, CaO:Ln, ZnS:Ln, Y₂O₃:Ln, oder MgF₂:Ln (Ln = Lanthaniden).

Die einfache Nachweissonde enthält lumineszierende anorganische dotierte Nanoteilchen (lad-Nanoteilchen), die nach Anregung mit einer Strahlungsquelle durch Absorption und/oder Streuung und/oder Beugung der Anregungsstrahlung oder durch Emission von Fluoreszenzlicht nachweisbar sind und deren Oberfläche so präpariert ist, dass Affinitätsmoleküle zum Nachweis einer biologischen oder sonstigen organischen Substanz an diese präparierte Oberfläche ankoppeln können.

Die Präparation der Oberfläche kann darin bestehen, dass die Oberfläche der lad-Nanoteilchen chemisch modifiziert ist und/oder reaktive Gruppen und/oder kovalent oder nicht kovalent gebundene Verbindungsmoleküle aufweist.

Als Beispiel einer chemischen Modifikation der Oberfläche des lad-Nanoteilchens sei die Umhüllung des lad-Nanoteilchens mit genannt: Silica ermöglicht eine einfache chemische Konjugation von organischen Molekülen, da Silica sehr leicht mit organischen Linkern wie z.B. Triethoxysilanen oder Chlorsilanen reagiert.

Eine weitere Möglichkeit der Präparation der Oberfläche der lad-Nanoteilchen besteht darin, die oxidischen Übergangsmetallverbindungen, aus denen die lad-Nanoteilchen bestehen, mit Chlorgas oder organischen Chlorierungsagenzien in die entsprechenden Oxichloride zu überführen. Diese Oxichloride reagieren ihrerseits mit Nukleophilen wie z.B. Aminogruppen unter Bildung von Übergangsmetallstickstoffverbindungen. Auf diese Weise lässt sich zum Beispiel über die Aminogruppen von Lysinseitenketten eine direkte Konjugation von Proteinen erzielen. Die Konjugation mit Proteinen nach der Oberflächenmodifikation mit Oxichloriden kann auch durch Verwendung eines bifunktionellen Linkers wie Maleimidopropionsäurehydrazid bewerkstelligt werden.

Für nicht-kovalente Verknüpfungen eignen sich dabei besonders kettenförmige Moleküle mit einer der Oberfläche des lad-Nanoteilchen entgegengesetzten Polarität oder Ladung. Als Beispiele für Verbindungsmoleküle, die nicht-kovalent mit den lad-Nanoteilchen verknüpft sind, seien anionische, kationische oder zwitterionische Detergenzien, saure oder basische Proteine, Polyamine, Polyamide oder Polysulfon- oder Polycarbonsäuren genannt. Die Adsorption dieser Moleküle auf die Oberfläche des lad-Nanoteilchens lässt sich durch einfache Koinkubation erzielen. Die Anbindung eines Affinitätsmoleküls an diese nicht-kovalent gebundenen Verbindungsmoleküle kann dann mit Standardmethoden der Organischen Chemie erfolgen wie Oxidation, Halogenierung, Alkylierung, Acylierung, Addition, Substitution oder Amidierung des adsorbierten oder adsorbierbaren Materials. Diese Methoden zur Anbindung eines Affinitätsmoleküls an das nicht-kovalent gebundene Verbindungsmolekül, kann vor Adsorption an das lad-Nanoteilchen auf das Verbindungsmolekül angewendet werden, oder nachdem es bereits an das lad-Nanoteilchen adsorbiert ist.

Nicht nur die Oberfläche der lad-Nanoteilchen kann reaktive Gruppen aufweisen, sondern auch die angebundenen Verbindungsmoleküle können ihrerseits auch reaktive Gruppen aufweisen, die als Anknüpfungspunkte an der Oberfläche des lad-Nanoteilchens oder an weiteren Verbindungsmolekülen oder Affinitätsmolekülen dienen können. Solche reaktiven Gruppen, die sowohl geladen, ungeladen oder mit Partialladungen versehen sein können, können sowohl an der Oberfläche der lad-Nanoteilchen als auch Teil der Verbindungsmoleküle sein. Mögliche reaktive funktionale Gruppen können Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Phosphonsäuren, Phosphorsäureester, Sulfonsäuren, Azolide oder Derivate der genannten Gruppen sein.

Auch Nukleinsäuremoleküle können als Verbindungsmoleküle dienen. Sie bilden die Verbindung zu einem Affinitätsmolekül, das seinerseits Nukleinsäuremoleküle mit zu den Verbindungsmolekülen komplementären Sequenzen enthält.

Gegenstand der vorliegenden Erfindung ist weiterhin die Bereitstellung einer erweiterten Nachweissonde, die aus einer Kombination der einfachen Nachweissonde mit einem oder mehreren Affinitätssmolekülen oder mehreren miteinander gekoppelten Affinitätsmolekülen besteht. Diese Affinitätsmoleküle bzw. die Kombination verschiedener Affinitätsmoleküle werden auf Basis ihrer spezifischen Affinität gegenüber der biologischen Substanz ausgewählt, um deren Präsenz oder Abwesenheit nachzuweisen zu können. Es kann dabei jedes Molekül oder jede Kombination von Molekülen als Affinitätsmoleküle verwendet werden, die sich einerseits an die einfachen Nachweissonden konjugieren lassen und andererseits spezifisch an die nachzuweisende biologische oder sonstige organische Substanz anbinden. Die einzelnen Bestandteile einer Kombination von Molekülen können gleichzeitig oder nacheinander auf die einfachen Nachweissonden aufgebracht werden.

Generell können solche Affinitätssmoleküle verwendet werden, wie sie auch bei den im Stand der Technik beschriebenen fluoreszierenden organischen Farbstoffmolekülen benutzt werden, um diese spezifisch an die nachzuweisende biologische oder sonstige organische Substanz zu binden. Ein Affinitätssmolekül kann ein monoklonaler oder polyklonaler Antikörper, ein anderes Protein, ein Peptid, ein Oligonukleotid, ein Plasmid oder ein anderes Nukleinsäuremolekül, ein Oligo- oder Polysaccharid oder ein Hapten, wie Biotin oder Digoxin oder ein niedermolekulares synthetisches oder natürliches Antigen sein. Eine Liste solcher Moleküle sind in der allgemein zugänglichen Literatur veröffentlicht, z. B. in "Handbook of Fluorescent Probes and Research Chemicals" (7^{th} edition, CD-ROM) von R. P. Hauglund, Molecular Probes, Inc..

Die Affinität der erweiterten Nachweissonde gegenüber dem nachzuweisenden biologischen Agens ist im allgemeinen dadurch gegeben, dass die einfache Nachweissonde mit einem - normalerweise organischen - Affinitätsmolekül gekoppelt ist, das die gewünschte Affinität gegenüber dem nachzuweisenden Agens besitzt. Dabei werden reaktive Gruppen auf der Oberfläche des Affinitätsmoleküls und der einfachen Nachweissonde genutzt, um diese beiden kovalent oder nicht-kovalent zu binden. Reaktive Gruppen auf der Oberfläche des Affinitätsmoleküls sind dabei Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbbonylverbindungen oder Azolide. Auf der Oberfläche der einfachen Nachweissonde können die weiter oben beschriebenen Gruppen zur Konjugation des Affinitätsmoleküls verwendet werden.

Als eine unter vielen Möglichkeiten, eine einfache Nachweissonde mit einem Protein als Affinitätsmolekül zu verknüpfen, sei die folgende Reaktion genannt. Ein Silikaüberzogenes lad-Nanoteilchen reagiert mit 3-Aminopropyltriethoxysilan (Pierce, Rockford, IL, USA) und es folgt anschließend eine SMCC-Aktivierung (Succinimidyl-4-[N-maleimidomethyl]-cyclohexan-1-carboxylat) (Pierce). Die für die Reaktion an dieses aktivierte lad-Nanoteilchen benötigten proteingebundenen Thiolgruppen können durch die Reaktion eines Lysin enthaltenden Proteins mit 2-Iminothiolan (Pierce) erzeugt werden. Dabei reagieren Lysinseitenketten des zu konjugierenden Proteins mit 2-Iminothiolan unter Ringöffnung und Thioamidinbildung. Die dabei gebildeten, kovalent mit dem Protein verknüpften Thiolgruppen vermögen dann in einer Hetero-Michael Addition mit den auf die Oberfläche der einfachen Nachweissonde konjugierten Maleimidgruppen reagieren, um eine kovalente Bindung zwischen Protein als Affinitätsmolekül und der einfachen Nachweissonde auszubilden.

Neben der oben genannten Möglichkeit, erweiterte Nachweissonden durch Kopplung aus Affinitätsmolekül und einfacher Nachweissonden zu bilden, gibt es zahllose andere Verfahren, die aus der bekannten Reaktivität zahlreicher käuflicher Linker-Moleküle ableitbar sind.

Außer kovalenten Verknüpfungen zwischen einfacher Nachweissonde und Affinitätsmolekül sind nicht-kovalente, selbst-organisierte Verbindungen realisierbar. Als eine Möglichkeit sei hierbei die Verknüpfung von einfachen Nachweissonden mit Biotin als Verbindungsmolekül mit Avidin- oder Streptavidin- gekoppelten Affinitätsmolekülen genannt.

Eine weitere nicht-kovalente selbst-organisierte Verbindung zwischen einfacher Nachweissonde und Affinitätsmolekül besteht in der Wechselwirkung von einfachen Nachweissonden enthaltend Nukleinsäuremoleküle mit komplementären Sequenzen, die auf die Oberfläche eines Affinitätsmoleküls konjugiert sind.

Eine erweiterte Nachweissonde kann auch dadurch gebildet werden, dass Nukleinsäuresequenzen direkt an die präparierte Oberfläche einer einfachen Nachweissonde gebunden sind oder die reaktive Gruppe eines Affinitätsmoleküls bilden. Eine solche erweiterte Nachweissonde wird für den Nachweis von Nukleinsäuremolekülen mit komplementären Sequenzen verwendet.

Gegenstand der vorliegenden Erfindung ist weiterhin ein Verfahren zur Herstellung einer einfachen Nachweissonde, ein Verfahren zur Herstellung der erweiterten Nachweissonde und ein Verfahren zum Nachweis einer bestimmten Substanz in einem biologischen Material.

Das erfindungsgemäße Verfahren zur Herstellung der einfachen Nachweissonde enthält die Schritte:
a) Herstellung von lad-Nanoteilchen
b) chemische Modifikation der Oberfläche der lad-Nanoteilchen und /oder
c) Herstellung reaktiver Gruppen auf der Oberfläche der lad-Nanoteilchen und/oder
d) Verbindung eines oder mehrerer Verbindungsmoleküle mit der Oberfläche der lad-Nanoteilchen durch kovalente oder nicht-kovalente Bindung.

Bevorzugt ist die Verteilungsbreite der Ausdehnungen der in Schritt a) hergestellten lad-Nanoteilchen auf einen Bereich von +/-20 % einer mittleren Ausdehnung eingeschränkt.

Das erfindungsgemäße Verfahren zur Herstellung der erweiterten Nachweissonde enthält die Schritte:
e) Bereitstellung der einfachen Nachweissonde
f) Modifikation der Oberfläche eines Affinitätsmoleküls, um reaktive Gruppen einzuführen, die eine Konjugation mit dem Verbindungsmolekül erlauben
g) Konjugation des aktivierten Affinitätsmoleküls und der einfachen Nachweissonde.

Das erfindungsgemäße Verfahren zum Nachweis einer bestimmten Substanz in einem biologischen Material enthält die Schritte:
h) Zusammenführen der erweiterten Nachweissonde und des biologischen und/oder organischen Materials
i) Abtrennung von erweiterten Nachweissonden, die keine Bindung eingegangen sind
j) Belichtung des Materials mit einer elektromagnetischen Strahlung oder mit einem Teilchenstrahl
k) Messung des Fluoreszenzlichtes oder Messung der Absorption und/oder Streuung und/oder Beugung der Strahlung oder der Änderung derselben.

Zum Nachweis eines Analyten in einem zu untersuchenden biologischen Material, wird die erweiterte Nachweissonde mit einem zu untersuchenden Material kontaktiert. Bei dem zu untersuchenden biologische Material kann es sich um Serum, Zellen, Gewebeschnitte, Rückenmarksflüssigkeit, Sputum, Plasma, Urin oder jede andere beliebige Probe humanen, tierischen oder pflanzlichen Ursprungs handeln.

Dabei sollte der zu untersuchende Analyt vorzugsweise bereits immobilisiert sein oder sich in einer simultan oder konsekutiv ablaufenden Bildung supermolekularer Verbände immobilisieren lassen. Beispiel für solche Immobilisierungen ist ein ELISA (Enzyme Linked Immuno Sorbent Assay) bei dem die zu detektierenden Antigene durch adsorbierte oder anderweitig gebundene Erstantikörper spezifisch an einer festen Phase angebunden sind. Eine Immobilisierung des zu detektierenden Antigens ist auch leicht realisierbar, wenn es in einem existierenden Zellverband, wie einem Gewebeschnitt, oder in auf einer Unterlage fixierten Einzelzellen enthalten ist.

Bringt man den derartig immobilisierten Analyten mit den erweiterten Nachweissonden in Kontakt, so werden diese über das in ihnen enthaltene Affinitätsmolekül spezifisch an den Analyten anbinden. Ein Überschuss an erweiterten Nachweissonden lässt sich leicht wegwaschen, und es verbleiben nur spezifisch gebundene erweiterte Nachweissonden in der zu untersuchenden Probe. Bei Bestrahlung der so vorbereiteten Probe mit einer geeigneten Energiequelle lässt sich die Anwesenheit der erweiterten Nachweissonde, die das lad-Teilchen enthält, durch die Detektion des emittierten Fluoreszenzlichtes oder durch die Messung von Änderungen in der absorbierten, gestreuten oder gebeugten Strahlung nachweisen. Damit wird das Vorhandensein derjenigen biologischen und/oder organischen Substanzen detektiert, die eine geeignete Affinität gegenüber der erweiterten Nachweissonde aufweisen. Auf diese Weise lassen sich Substanzen unabhängig von ihrer chemischen Natur in einem Assay qualitativ und quantitativ nachweisen, solange ein anderes Molekül mit einer genügend hohen Affinität ihnen gegenüber existiert. Die erweiterten Nachweissonden sind dadurch spezifisch, dass an der Oberfläche der in ihnen enthaltenen einfachen Nachweissonden ein solches Affinitätssmolekül angebunden ist, das eine hohe spezifische Bindungskonstante mit der nachzuweisenden biologischen Substanz besitzt. Auf diese Weise lassen sich auch bestimmte Zelltypen (zum Beispiel Krebszellen) nachweisen. Dabei können zelltypspezifische Biomoleküle, auf der Zelloberfläche oder auch im Inneren der Zelle mit den Nachweissonden markiert und optisch über ein Mikroskop oder über ein Flow Cytometer nachgewiesen werden.

Nach dem oben beschriebenen Nachweisprinzip können auch mehrere verschiedene Analyte gleichzeitig in einem biologischen und/oder organischen Material nachgewiesen werden (Multiplexing). Dies geschieht indem das zu untersuchende biologische und/oder organische Material gleichzeitig mit verschiedenen Nachweissonden in Verbindung gebracht wird. Die verschiedenen Nachweissonden unterscheiden sich dadurch voneinander, dass ihre Affinitätsmoleküle an verschiedene Analyte anbinden und die in ihnen enthaltenen lad-Nanoteilchen bei verschiedenen Wellenlängen absorbieren, streuen, beugen oder Fluoreszenzlicht emittieren.

Die erfindungsgemäße Nachweissonde ist stabil gegenüber der eingestrahlten Energie sowie stabil gegenüber Sauerstoff oder Radikalen. Das Material, aus dem die erfindungsgemäßen Nachweissonden bestehen, ist nicht-toxisch oder nur gering toxisch. Eine sehr enge Verteilungsbreite der Größe der lad-Nanoteilchen ist nicht notwendig, da die spektrale Lage der Fluoreszenzbanden und deren Bandbreiten von der Dotierung und nicht wesentlich von der Größe der lad-Nanoteilchen abhängt. Ebenso ist keine anorganische Hülle um die Teilchen zur Stabilisierung der Fluoreszenzausbeute erforderlich. Sie kann jedoch zur Erleichterung der Konjugationschemie verwandt werden. Ein weiterer Vorteil ist, dass die Anregung durch eine einzige breit- oder schmalbandige Strahlungsquelle erfolgen kann, da die Absorptionswellenlänge der anregenden Strahlung bzw. die Anregungswellenlänge der Teilchen nicht mit der Emissionswellenlänge korreliert ist. Außerdem erlaubt eine zeitaufgelöste Fluoreszenzmessung die Trennung des spezifischen Fluoreszenzlichtes von unspezifischer Hintergrundfluoreszenz, da die Lebensdauer des durch die äußere Strahlungsquelle angeregten Zustandes in dem lad-Teilchen, der dann zur Lichtemission führt, meist wesentlich länger ist als die der Hintergrundfluoreszenz.

Die Verwendung der erfindungsgemäßen Nachweissonde und des erfindungsgemäßen Verfahrens liegt vorzugsweise in der medizinischen Diagnostik und in der Screeningtechnik, insbesondere dort, wo die Markierung spezifischer Substanzen zum Zwecke ihres Nachweises, ihrer Lokalisierung und/oder ihrer Quantifizierung eine besondere Rolle spielt. Dazu zählen der Nachweis spezifischer Antikörper in diagnostischen Assays, die mit Blut oder anderen Körpermaterialien durchgeführt werden. Die erfindungsgemäßen Nachweissonden können aber auch in der zellulären Analytik verwendet werden, das heißt zum Nachweis spezieller Zellen, wie Krebszellen. Bei den genannten Verwendungsmöglichkeiten bieten die erfindungsgemäßen Nachweissonden besondere Vorteile, da hier die Möglichkeit des Multiplexing, also der simultane Nachweis verschiedener Antigene in einem Assay oder sogar in einer einzigen Zelle, ausgenutzt werden kann.

### Beispiele

### Beispiel 1: Die Herstellung von lad-Nanoteilehen bestehend aus YVO₄:Ln

Der erste Schritt besteht in der Bereitstellung von YVO₄:Ln. YVO₄:Ln kann nach dem in K. Riwotzki, M. Haase; Journal of Physical Chemistry B; Vol. 102, 1998, Seite 10130, linke Spalte angegebenen Verfahren hergestellt werden. In einem Teflonbehälter wird 3,413 g Y(NO₃)₃·6H₂O (8,9 mmol) und 0,209 g Eu(NO₃)₃·6H₂O (0,47 mmol) in 30 ml destilliertem Wasser gelöst. Dazu wird unter Rühren 2,73 g Na₃(VO₄)·10H₂O, das in 30 ml destilliertem Wasser gelöst wurde, gegeben. Nach 20 min weiterem Rühren wird der Teflonbehälter in einen Autoklaven gestellt und unter weiterem Rühren auf 200°C erhitzt. Nach 1h wird die Dispersion aus dem Autoklaven entnommen und bei 3000g 10 min zentrifugiert. Der Feststoffanteil wird extrahiert und in 40 ml destilliertem Wasser aufgenommen. 3220 g einer wässrigen 1-Hydroxyethan-1,1-Diphophonsäure-Lösung (60 Gew.-%) wird zu der Dispersion gegeben (9,38 mmol). Zur Entfernung von Y(OH)₃, das sich aus überschüssigen Yttriumionen gebildet hat, wird der pH-Wert mit HNO₃ auf 0,3 eingestellt und 1h gerührt. Dabei bildet sich kolloidales V₂O₅, das sich durch eine rötliche Färbung der Lösung bemerkbar macht. Danach wird der pH-Wert mit NaOH auf 12,5 eingestellt und über Nacht in einem geschlossenen Behälter gerührt. Die resultierende weiße Dispersion wird dann bei 3000g für 10 min. zentrifugiert und der Überstand mit seinen Nebenprodukten entfernt. Der Niederschlag besteht aus YVO₄:Eu und kann mit 40 ml destilliertem Wasser aufgenommen werden.

Zur Isolation der Nanoteilchen, die kleiner als ca. 30 nm sind, wird die Dispersion bei 3000g für 10 min. zentrifugiert, der Überstand dekantiert und zurückgestellt. Anschließend wurde der Niederschlag noch einmal mit 40 ml destilliertem Wasser aufgenommen, bei 3000g für 10 min. zentrifugiert und der Überstand dekantiert. Dieser und der zurückgestellte Überstand wurden zusammen gegeben und bei 60000g 10 min zentrifugiert. Der hieraus resultierende Überstand enthält die gewünschten Teilchen. Nach einem weiteren Dialyseschritt (Dialyseschlauch Serva, MWCO 12 - 14 kD) erhält man eine kolloidale Lösung, aus der durch Trocknung mit einem Rotationsverdampfer (50°C) ein redispergierbares Pulver gewonnen werden kann.

### Beispiel 2: Die Herstellung von lad-Nanoteilchen bestehend aus LaPO₄:Eu

Der erste Schritt besteht in der Bereitstellung von LaPO₄:Eu. LaPO₄:Eu kann nach dem in H. Meyssamy, K. Riwotzki, A. Kornowski, S. Naused, M. Haase; Advanced Materials, Vol. 11, Issue 10, 1999, Seite 843, rechte Spalte unten bis Seite 844, linke Spalte oben angegebenen Verfahren hergestellt werden. In einem Teflontopf wird 12,34 g La(NO₃)₃·6H₂O (28,5 mmol) und 0,642 g Eu(NO₃)₃·5H₂O (1,5 mmol) in 50 ml destilliertem Wasser gelöst und in 100 ml NaOH (1 M) gegeben. Dazu wird unter Rühren eine Lösung aus 3,56 g (NH₄)₂HPO₄ (27 mmol) in 100 ml destilliertem Wasser gegeben. Die Lösung wird mit NaOH (4 M) auf pH 12,5 eingestellt und in einem Autoklaven 2 h bei 200°C unter kräftigem Rühren erhitzt. Die Dispersion wird dann bei 3150g 10 min. zentrifugiert und der Überstand entfernt. Um unerwünschtes La(OH)₃ zu entfernen, wird der Niederschlag in HNO₃ (1M) dispergiert und 3 Tage gerührt (pH 1). Danach wird die Dispersion zentrifugiert (3150g, 5 min) und der Überstand entfernt. Dem Zentrifugat wird unter Rühren 40 ml destilliertes Wasser zugesetzt.

Die milchige Dispersion enthält noch eine breite Größenverteilung. Zur Isolation der Nanoteilchen, die kleiner als ca. 30 nm sind, werden in vollständiger Analogie zu Beispiel 1 entsprechende Zentrifugations- und Dekantierungsschritte nachgeschaltet.

### Beispiel 3: Die Herstellung von lad-Nanoteilchen bestehend aus LaPO₄:Ce, Tb

Der erste Schritt besteht in der Bereitstellung von LaPO₄:Ce, Tb. 300 ml Trisethylhexylphosphat werden in einem trockenen Stickstoff-Gasstrom gespült. Anschließend werden 7,43 g LaCl₃•7H₂O (20 mmol), 8,38 g CeCl₃•7H₂O (22,5 mmol) und 2,8 g TbCl₃•6H₂O (7,5 mmol) in 100 ml Methanol gelöst und hinzugegeben. Danach wird Wasser und Methanol durch Heizen der Lösung auf 30°C bis 40°C im Vakuum abdestilliert. Eine frisch hergestellte Lösung bestehend aus 4,9 g kristalliner Phosphorsäure (50 mmol), die in einer Mischung aus 65,5 ml Trioctylamin (150 mmol) und 150 ml Trisethylhexylphosphat gelöst wurden, werden anschließend hinzugegeben. Die klare Lösung muss rasch in ein zu evakuierendes Gefäß gestellt und mit Stickstoff-Gasstrom gespült werden, um die Oxidation von Ce³⁺ bei einer Temperaturerhöhung zu minimieren. Anschließend wird die Lösung auf 200°C erhitzt. Während der Heizphase werden einige der Phosphorsäureester-Gruppen gespalten, was zu einer allmählichen Erniedrigung der Siedepunktes führt. Die Heizphase wird beendet, wenn die Temperatur auf 175°C fällt (ca. 30 bis 40 h). Nachdem die Lösung auf Zimmertemperatur abgekühlt ist, wird ein 4-facher Überschuss an Methanol hinzugegeben, was zu einer Ausfällung der Nanoteilchen führt. Der Niederschlag wird abgetrennt, mit Methanol gewaschen und getrocknet.

### Beispiel 4: Die Herstellung von lad-Nanoteilchen bestehend aus LaPO₄:Eu

490 mg (5,0 mmol) kristalline Phosphorsäure und 6,5 ml (15 mmol) Trioctylamin werden in 30 ml Trisethylhexlphosphat gelöst. Anschließend werden 1,76 g La(NO₃)₃•7H₂O (4,75 mmol) und 92 mg EuCl₃•6H₂O (0,25 mmol) in 50 ml Trisethylhexlphosphat gelöst und mit der ersten Lösung vereint. Die resultierende Lösung wird unter Vakuum entgast und anschließend für 16 h unter Stickstoff bei 200°C geheizt. Während der Heizphase werden einige der Phosphorsäureester-Gruppen gespalten, was zu einer allmählichen Erniedrigung der Siedepunktes führt. Die Heizphase wird beendet, wenn die Temperatur auf 180°C fällt. Nachdem die Lösung auf Zimmertemperatur abgekühlt ist, wird Methanol hinzugegeben, was zu einer Ausfällung der Nanoteilchen führt. Der Niederschlag wird mit Hilfe einer Zentrifuge abgetrennt, mit Methanol zweimal gewaschen und getrocknet.

### Beispiel 5: Umhüllung der in Beispiel 2 dargestellten Nanopartikel mit einer SiO₂-Hülle

1 g der nach Beispiel 2 hergestellten LaPO₄:Eu-Nanoteilchen werden unter intensivem Rühren mit einem Magnetrührer bei 900 Upm in 100 ml Wasser gegeben und mit Tetrabutylammoniumhydroxid auf einen pH-Wert von 12 eingestellt. Die Dispersion wird dann unter intensivem Rühren mit 500 mg Natrium-Wasserglas (26,9% SiO₂; 8,1 % Na20) versetzt. Anschließend werden ebenfalls unter intensivem Rühren tropfenweise 50 ml Ethanol hinzugegeben. Der entstandene Niederschlag wird abzentrifugiert und der Rückstand in 50 ml deionisiertem Wasser im Ultraschallbad redispergiert. Die Dispersion wird dann durch Dekantieren vom nicht dispergierten Anteil getrennt und mit Hilfe eines Rotationsverdampfers bei einem Druck von 10 mbar und einer Temperatur von 80°C getrocknet.

### Beispiel 6: Die Konjugation von den in Beispiel 5 dargestellten Nanopartikeln mit anti-α-Aktin Antikörpern

100 mg der in Beispiel 5 synthetisierten Silika-umhüllten Nanoteilchen werden in 10 mL trockenem Tetrahydrofuran (THF) dispergiert und mit 100 µL N-Methylmorpholin versetzt. Zu der Lösung gibt man 0.5 mL einer 10%igen Lösung von 3-Aminopropyltriethoxysilan in THF. Die Lösung wird im fest verschlossenen Gefäß über Nacht bei 40°C gerührt. Die Lösung wird mit 5 mL Wasser versetzt, 1 h bei Raumtemperatur nachgerührt und mit einer Glassfritte (4G, Schott) von eventuell ausgefallenem Material abfiltiert. Das Filtrat wird in Utrafiltrationsröhrchen (Centricon, Amicon, Ausschlussgröße 10, kD) in TSE7-Puffer umgepuffert. (TSE7: 100 mmol Triethanolamin, 50 mmol Natriumchlorid, 1 mmol EDTA in Wasser, pH 7.3). Das Zielvolumen beträgt 2 mL, der Austauschfaktor 1000. Die von der Ultrafiltrationsmembran zurückgehaltenen amino-aktivierten Nanopartikel in 2 mL TSE7 Puffer werden mit 500 µL einer 20 mmol Lösung von sSMCC (Sulfosuccimidyl-4-[N-maleimidomethyl]cyclohexancarboxylat (Pierce, Rockwell, IL, USA) versetzt und 60 Minuten bei 25°C gerührt. Die erhaltene Mischung wird wie oben beschrieben in 10 kD Centriconröhrchen in TSE7 Puffer umgepuffert und auf 2 mL eingeengt. Dieser Schritt wird bei 5°C durchgeführt. Die erhaltene Lösung ist im Kühlschrank 12 h stabil. 10 mg monoclonaler anti Aktin Antikörper (Sigma) werden mit Centricon Ultrafiltrationsröhrchen (Ausschlussgröße 50 kD) in TSE8 Buffer überführt (Austauschfaktor 1000, TSE8: (100 mmol Triethanolamin, 50 mmol Natriumchlorid, 1 mmol EDTA in Wasser, pH 8.5). Die Proteinkonzentration wird auf 7-8 mg/mL eingestellt. Zu der Antikörperlösung gibt man 150 µL einer 10 mM Lösung von 2-Imminothiolan in TSE8 Puffer und lässt die Mischung 15 Minuten reagieren. Der so Thiol-aktivierte Antikörper wird wie oben beschrieben bei 4°C mit TSE8 Puffer umgepuffert, um nicht reagierte Aktivatormoleküle zu entfernen und auf 2 mL eingeengt. Die aktivierten Nanopartikel und den aktivierten Antikörper enthaltenden Lösungen werden vereinigt und bei Raumtemperatur über Nacht gerührt. Die so erhaltene Dispersion des erweiterten Nachweisteilchen wird von nicht umgesetztem Antikörper durch Gelmermeationschromatographie auf Superdex 200 (Pharmacia) gereinigt. Als Laufpuffer wird TSE7 verwendet. Die Retentionszeit für den unkonjugierten erweiterten lad-Nanopartikel beträgt etwa 2 Stunden.

### Beispiel 7: Die Konjugation von in Beispiel 1 dargestellten lad-Nanopartikeln mit anti Myoglobin Antikörpern

100 mg der in Beispiel 1 dargestellten Vanadat-Nanopartikel werden in einem Glasrohr im Argonstrom mit einem Heizband erhitzt. Nach Ausheizen der Partikel für etwa eine Stunde werden dem Gasstrom für etwa 3 min 3-5 Vol.% Chlorgas zudosiert. Die erforderliche Reaktionszeit hängt wesentlich von der Partikelgröße ab und sollte daher eventuell mit derselben Charge von Nanopartikeln austitriert werden. Man lässt die Partikel im Argonstrom abkühlen und gibt die partiell chlorierten Nanopartikel in 5 mL einer 50 mM Lösung von N-Maleimidopropionsäurehydrazid (Pierce) und rührt über Nacht bei 20°C. Die so erhaltene Lösung wird am Rotationsverdampfer bei Raumtemperatur eingeengt und wie in Beispiel 6 beschrieben mit Ultrafiltrationsröhrchen in TSE7 umgepuffert. Die erhaltene Dispersion ist bei 5°C 12 h stabil. 10 mg polyklonaler Kaninchen anti-Myoglobin Antikörper (Dako) werden mit Centricon Ultrafiltrationsröhrchen (Ausschlussgröße 50 kD) in TSE8 Buffer überführt (Austauschfaktor 1000, TSE8: (100 mmol Triethanolamin, 50 mmol Natriumchlorid, 1 mmol EDTA in Wasser, pH 8.5). Die Proteinkonzentration wird auf 7-8 mg/mL eingestellt. Zu der Antikörperlösung gibt man 150 µL einer 10 mM Lösung von 2-Imminothiolan in TSE8 Puffer und lässt die Mischung 15 Minuten reagieren. Der so Thiol-aktivierte Antikörper wird wie oben beschrieben bei 4°C mit TSE8 Puffer umgepuffert, um nicht reagierte Aktivatormoleküle zu entfernen und auf 2 mL eingeengt. Die aktivierten Nanopartikel und den aktivierten Antikörper enthaltenden Lösungen werden vereinigt und bei Raumtemperatur über Nacht gerührt. Die so erhaltene Dispersion des erweiterten Nachweisteilchen wird von nicht umgesetztem Antikörper durch Gelmermeationschromatographie auf Superdex 200 (Pharmacia) gereinigt. Als Laufpuffer wird TSE7 verwendet. Die Retentionszeit für den erweiterten lad-Nanopartikel beträgt etwa 2 Stunden.

### Beispiel 8: Visualisierung von Aktin Filamenten in Kaninchen Muskelzellen durch Nanopartikel Fluoreszenz

Ein mit einem Gefriermicroton geschnittener Dünnschnitt eines Kaninchenmuskels wird auf einen Objektträger aufgebracht und für 3 Minuten in eiskalten Ethanol fixiert. Der auf den Objektträger aufgebrachte Dünnschnitt wird anschließend zwei Mal mit PBS-Tween Buffer (137 mM NaCl, 2.7 mM KCl,10.1 mM Na2HPO4, 1.8 mM, KH2P04, 0.1 % Tween 20) gewaschen und dabei für jeweils 5 min in dem Waschpuffer belassen. Zur Verringerung der nicht spezifischen Bindung wird der Dünnschnitt 30 min bei 20°C in einer Lösung von 1.5 % Schafserum in PBS-Tween Puffer inkubiert und der Dünnschnitt wie oben beschrieben zwei Mal gewaschen. Die in Beispiel 6 beschriebene Lösung des erweiterten Nachweisteilchens wird 1: 100 mit PBS-S (137 mM NaCl, 2.7 mM KCl, 10.1 mM Na2HP04, 1.8 mM, KH2P04, 0.1 % Tween 20, 1.5% Schafserum) verdünnt und der Dünnschnitt in dieser Lösung bei 20°C eine Stunde inkubiert. Nach Beendigung der Inkubation wird sie wie oben beschrieben gewaschen. Die Detektion erfolgt nach Anregung mit einem Argon Ionen Laser bei Wellenlängen zwischen 333 nm und 364 nm durch Messung des Fluoreszenzlichts bei einer Wellenlänge von 591 nm. Benutzt wurde hierfür ein Konfokales Laserscanning Mikroskop der Firma Leica, Typ TCS NT.

### Beispiel 9: Nachweis von Myoglobin in humanem Serum durch Nanopartikel Fluoreszenz

Monoklonaler anti Myoglobin Antikörper (BiosPacific) werden in einer Konzentrations von 5 mg/L in C Puffer gelöst (100 mmol Natriumcarbonat in Wasser, pH 9.0. Jeweils 200 µL dieser Lösung werden in 96 Wells einer Standard Polystyrol ELISA Platte (Greiner) pipettiert, die Platte versiegelt und 2 h bei 37°C inkubiert. Die Platte wird ausgeklopft und mit 200 µL einer 1% BSA Lösung (Bovines Serum Albumin) in TSE7 Puffer (siehe Beispiel 6) geblockt. Man wäscht drei mal mit je 250 µL TSET7 Puffer (100 mmol Triethanolamin, 50 mmol Natriumchlorid, 1 mmol EDTA, 0.1% Tween 20 in Wasser, pH 7.3. Man pipettiert jeweils 100 µL eines 6-Level Myoglobin Kalibrators (Bayer Immuno 1) und Humanseren in die unterschiedlichen Wells der Mikrotiterplatte und lässt 2h bei Raumtemperatur inkubieren. Die Analytlösungen werden aus der Platte aus pipettiert und die Platte drei mal wie oben beschrieben gewaschen. Zu jedem Well gibt man etwa 1 µg der in Beispiel 7 dargestellten erweiterten anti Myoglobin Nachweissonde, dispergiert in 100 µL TSE7. Man lässt 1 h bei Raumtemperatur inkubieren, wäscht drei mal mit TSET7. Der Readout des ELISAs erfolgt durch Messung der lad-Nanopartikelfluoreszenz in einem Mikrotiterplattenleser (Tecan).

### Beispiel 10: Lösen der in Beispiel 3 dargestellten Nanopartikel in Wasser durch Umsetzung von Ethylen- oder Polyethylenglykol

1 g der in Beispiel 3 hergestellten LaPO₄:Ce,Tb (~5 mMol) werden mit 100 ml Ethylenglykol (~2 Mol) (alternativ können auch Polyethylenglykole verschiedener Kettenlänge, HO-(CH₂-CH₂-O)ₙ-OH, wobei n = 2 - 9, verwendet werden) und 100 mg Paratoluolsulfonsäure unter Rühren und Stickstoff auf 200°C erhitzt. Dabei lösen sich die Partikel und bleiben auch nach Abkühlen auf RT in Lösung. Anschließend wird gegen Wasser über Nacht dialysiert (cut off MW 10-20.000).

### Beispiel 11: Funktionalisierung von in Beispiel 10 hergestellten Nanopartikeln durch Oxidation

Zu 100mg (0,5 mMol in 20 ml Wasser) der in Beispiel 10 dargestellten Nanopartikel wird unter Rühren zunächst 0.5 mL 96-98%ige Schwefelsäure dazugegeben. Tropfenweise wird 1mM KMnO₄ Lösung dazugegeben. bis keine Entfärbung der violetten Farbe mehr auftritt. Man gibt anschließend nochmals dieselbe Menge KMnO₄- Lösung hinzu und läßt über Nacht bei Raumtemperatur nachrühren (>12 h). Überschüssiges Permanganat wird durch tropfenweise Zugabe von frisch bereiteter 1mM Natriumsulfit Lösung reduziert. Nacht wird gegen 0,1M MES, 0,5M NaCl, pH 6,0 dialysiert.

### Beispiel 12: Die Konjugation von in Beispiel 11 dargestellten Nanopartikein an anti-Biotin Antikörper

1mg (5nMol) der in Beispiel 11 dargestellten carboxy-funktionalisierten Nanopartikel in 1mL Puffer (0.1 M MES, 0.5 M NaCl, pH 6) werden 0,4 mg EDC (~2mM) und 1,1 mg (~5mM) sulfo-NHS (beide Pierce; Rockford, IL) gegeben und für 15 min bei RT gerührt. Das nicht umgesetzte EDC wird durch Zugabe von 1,4µl 2-Mercaptoethanol (Endkonzentration 20mM) inaktiviert. Polyklonaler Ziege anti.Biotin Antikörper (Sigma) wird in der gleichen molaren Menge (5nMol) in Aktivierungs- Puffer (0,1M MES, 0,5M NaCl, pH 6,0) hinzugefügt und das Gemisch für 2 Std. bei RT gerührt. Die Reaktion wird durch Zugabe von Hydroxylamin (Endkonzentration von 10mM) gestoppt. Die so erhaltene Lösung der erweiterten Nachweisteilchen wird von nicht umgesetzten Antikörper durch Gelmermeationschromatographie auf Superdex 200 (Pharmacia) gereinigt. Als Laufpuffer wird Aktivierungs-Puffer verwendet. Die Retentionszeit für den erweiterten lad-Nanopartikel beträgt etwa 2 Stunden.

## Patentansprüche

1. Einfache Nachweissonde enthaltend lumineszierende anorganische dotierte Nanoteilchen (lad-Nanoteilchen), die nach Anregung mit einer Strahlungsquelle durch Absorption und/oder Streuung und/oder Beugung der Anregungsstrahlung oder durch Emission von Fluoreszenzlicht nachweisbar sind und deren Oberfläche so präpariert ist, dass Affinitätsmoleküle zum Nachweis einer biologischen oder sonstigen organischen Substanz an diese präparierte Oberfläche ankoppeln können, **dadurch gekennzeichnet, dass** die lad-Nanoteilchen Durchmesser im Bereich von 2 nm bis unter 20 nm aufweisen und dass als Verbindungsmolekül ein oder mehrere kettenförmige Moleküle mit einer der Oberfläche des lad-Nanoteilchens entgegengesetzten Polarität oder Ladung nicht-kovalent mit der Oberfläche der lad-Nanoteilchen verbunden sind.

2. Einfache Nachweissonde nach Anspruch 1, **dadurch gekennzeichnet, dass** die Oberfläche der lad-Nanoteilchen chemisch modifiziert ist und/oder reaktive Gruppen aufweist.

3. Einfache Nachweissonde nach Anspruch 1 oder 2, **dadurch gekennzeichnet, dass** die lad-Nanoteilchen von einer Schicht Silika umgeben sind.

4. Einfache Nachweissonde nach einem der Ansprüche 1 bis 3, **dadurch gekennzeichnet, dass** die Oberfläche der lad-Nanoteilchen Oxichloride aufweist, die durch die Behandlung von lad-Nanoteilchen bestehend aus oxidischen Übergangsmetallverbindungen mit Chlorgas oder organischen Chlorierungsagenzien entstehen.

5. Einfache Nachweissonde nach einem der Ansprüche 1 bis 4, **dadurch gekennzeichnet, dass** die kettenförmigen Moleküle anionische, kationische oder zwitterionische Detergenzien, saure oder basische Proteine, Polyamine, Polyamide oder Polysulfon- oder Polycarbonsäuren sind.

6. Einfache Nachweissonde nach einem der Ansprüche 1 bis 5, **dadurch gekennzeichnet, dass** die Oberfläche und/oder die mit der Oberfläche des lad-Nanoteilchens verbundenen Verbindungsmoleküle reaktive neutrale, geladene oder partialgeladene Gruppen wie Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Azolide, Phosphonsäuren, Phosphorsäureester oder Derivate der genannten Gruppen tragen, wobei diese reaktiven Gruppen eine chemische Bindung mit weiteren Verbindungsmolekülen oder Affinitätsmolekülen erlauben.

7. Einfache Nachweissonde nach einem der Ansprüche 1 bis 6, **dadurch gekennzeichnet, dass** Nukleinsäuremoleküle als Verbindungsmoleküle für ein Affinitätsmolekül enthaltend Nukleinsäuremoleküle mit zu den Verbindungsmolekülen komplementären Sequenzen dient.

8. Einfache Nachweissonde nach einem der Ansprüche 1 bis 7, **dadurch gekennzeichnet, dass** die Strahlungsquelle eine Quelle für elektromagnetische Strahlung mit Wellenlängen im Bereich des infraroten Lichts, des sichtbaren Lichts, des UV, des Röntgenlichts oder der γ-Strahlung ist oder eine Quelle für Teilchenstrahlung wie Elektronenstrahlung ist.

9. Einfache Nachweissonde nach einem der Ansprüche 1 bis 8, **dadurch gekennzeichnet, dass** die lad-Nanoteilchen eine nadelförmige Morphologie aufweisen mit einer Breite von 3 nm bis 50 nm, bevorzugt von 3 nm bis unter 20 nm und einer Länge von 20 nm bis 5 µm, bevorzugt von 20 nm bis 500 nm.

10. Einfache Nachweissonde nach einem der Ansprüche 1 bis 9, **dadurch gekennzeichnet, dass** das Wirtsmaterial der lad-Nanoteilchen Verbindungen des Typs XY enthält, wobei X ein Kation aus einem oder mehreren Elementen der Hauptgruppen 1a, 2a, 3a, 4a, der Nebengruppen 2b, 3b, 4b, 5b, 6b, 7b oder der Lanthaniden des Periodensystems ist und Y entweder ein mehratomiges Anion aus einem oder mehreren Element(en) der Hauptgruppen 3a, 4a, 5a, der Nebengruppen 3b, 4b, 5b, 6b, 7b und/oder 8b sowie Element(en) der Hauptgruppen 6a und/oder 7 oder ein einatomiges Anion aus der Hauptgruppe 5a, 6a oder 7a des Periodensystems ist.

11. Einfache Nachweissonde nach Anspruch 10, **dadurch gekennzeichnet, dass** das Wirtsmaterial der lad-Nanoteilchen Verbindungen aus der Gruppe der Sulfide, Selenide, Sulfoselenide, Oxysulfide, Borate, Aluminate, Gallate, Silikate, Germanate, Phosphate, Halophosphate, Oxide, Arsenate, Vanadate, Niobate, Tantalate, Sulfate, Wolframate, Molybdate, Alkalihalogenide sowie andere Halogenide oder Nitride enthält.

12. Einfache Nachweissonde nach einem der Ansprüche 1 bis 11, **dadurch gekennzeichnet, dass** als Dotierung ein oder mehrere Elemente aus der Gruppe enthaltend Elemente der Hauptgruppen 1a, 2a oder Al, Cr, Tl, Mn, Ag, Cu, As, Nb, Nd, Ni, Ti, In, Sb, Ga, Si, Pb, Bi, Zn, Co und/oder Elemente der Lanthaniden verwendet werden.

13. Einfache Nachweissonde nach Anspruch 12, **dadurch gekennzeichnet, dass** auch Kombinationen von zwei oder mehreren dieser Elemente in unterschiedlichen relativen Konzentrationen zueinander als Dotierungsmaterial dienen.

14. Einfache Nachweissonde nach einem der Ansprüche 1 bis 13, **dadurch gekennzeichnet, dass** die Konzentration des Dotierungsmaterials im Wirtsgitter zwischen 10⁻⁵ mol% und 50 mol%, bevorzugt zwischen 0,01 mol% und 30 mol%, besonders bevorzugt zwischen 0,1 mol% und 20 mol% beträgt.

15. Einfache Nachweissonde nach einem der Ansprüche 1 bis 14, **dadurch gekennzeichnet, dass** LiI:Eu; NaI:Tl; CsI:Tl; CsI:Na; LiF:Mg; LiF:Mg,Ti; LiF:Mg,Na; KMgF₃:Mn; Al₂O₃:Eu; BaFCI:Eu; BaFCI:Sm; BaFBr:Eu; BaFCl_{0,5}Br_{0,5}:Sm; BaY₂F₈:A (A = Pr, Tm, Er, Ce); BaSi₂O₅:Pb; BaMg₂Al₁₆O₂₇:Eu; BaMgAl₁₄O₂₃:Eu; BaMgAl₁₀O₁₇:Eu; BaMgAl₂O₃:Eu; Ba₂P₂O₇:Ti; (Ba,Zn,Mg)₃Si₂O₇:Pb; Ce(Mg,Ba)Al₁₁O₁₉; Ce_{0,65}Tb₀,₃₅MgAl₁₁O₁₉:Ce,Tb; MgAl₁₁O₁₉:Ce,Tb; MgF₂:Mn; MgS:Eu; MgS:Ce; MgS:Sm; MgS:(Sm,Ce); (Mg,Ca)S:Eu; MgSiO₃:Mn; 3,5MgO·0,5MgF₂·GeO₂:Mn; MgWO₄:Sm; MgWO₄:Pb; 6MgO·As₂O₅:Mn; (Zn,Mg)F₂:Mn; (Zn₄Be)SO₄:Mn; Zn₂SiO₄:Mn; Zn₂SiO₄:Mn,As; ZnO:Zn; ZnO:Zn,Si,Ga; Zn₃(PO₄)₂:Mn; ZnS:A (A = Ag, Al, Cu); (Zn,Cd)S:A (A = Cu, Al, Ag, Ni); CdBO₄:Mn; CaF₂:Mn; CaF₂:Dy; CaS:A A = Lanthanide, Bi); (Ca,Sr)S:Bi; CaWO₄:Pb; CaWO₄:Sm; CaSO₄:A (A = Mn, Lanthanide); 3Ca₃(PO₄)₂·Ca(F,Cl)₂:Sb,Mₙ; CaSiO₃:Mn,Pb; Ca₂Al₂Si₂O₇:Ce; (Ca,Mg)SiO₃:Ce; (Ca,Mg)SiO₃:Ti; 2SrO·6(B₂O₃)·SrF₂:Eu; 3Sr₃(PO₄)₂·CaCl₂:Eu; A₃(PO₄)₂·ACl₂:Eu (A = Sr, Ca, Ba); (Sr,Mg)₂P₂O₇:Eu; (Sr,Mg)₃(PO₄)₂:Sn; SrS:Ce; SrS:Sm,Ce; SrS:Sm; SrS:Eu; SrS:Eu,Sm; SrS:Cu,Ag; Sr₂P₂O₇:Sn; Sr₂P₂O₇:Eu; Sr₄Al₁₄O₂₅:Eu; SrGa₂S₄:A (A = Lanthanide, Pb); SrGa₂S₄:Pb; Sr₃Gd₂Si₆O₁₈:Pb,Mn; YF₃:Yb,Er; YF₃:Ln (Ln = Lanthanide); YLiF₄:Ln (Ln = Lanthanide); Y₃Al₅O₁₂:Ln (Ln = Lanthanide); YAl₃(BO₄)₃:Nd,Yb; (Y,Ga)BO₃:Eu; (Y, Gd)BO₃:Eu; Y₂Al₃Ga₂O₁₂:Tb; Y₂SiO₅:Ln (Ln = Lanthanide); Y₂O₃:Ln (Ln = Lanthanide); Y₂O₂S:Ln (Ln = Lanthanide); YVO₄:A (A = Lanthanide, In); Y(P, V)O₄:Eu; YTaO₄:Nb; YAlO₃:A (A = Pr, Tm, Er, Ce); YOCl:Yb, Er; LnPO₄:Ce, Tb (Ln = Lanthanide oder Mischungen von Lanthaniden) ; LuVO₄:Eu; GdVO₄:Eu; Gd₂O₂S:Tb; GdMgB₅O₁₀:Ce, Tb; LaOBr:Tb; La₂O₂S:Tb; LaF₃:Nd, Ce; BaYb₂F₈:Eu; NaYF₄:Yb, Er; NaGdF₄:Yb, Er; NaLaF₄:Yb, Er; LaF₃:Yb,Er,Tm; BaYF₅:Yb,Er; Ga₂O₃:Dy; GaN:A (A = Pr, Eu, Er, Tm); Bi₄Ge₃O₁₂; LiNbO₃:Nd, Yb; LiNbO₃:Er; LiCaAlF₆:Ce; LiSrAlF₆:Ce; LiLuF₄:A (A = Pr, Tm, Er, Ce); Li₂B₄O₇:Mn, SiOₓ:Er, Al (0 ≤ x ≤ 2) als Material für die lad-Nanoteilchen verwendet wird.

16. Einfache Nachweissonde nach einem der Ansprüche 1 bis 14, **dadurch gekennzeichnet, dass** YVO₄:Eu, YVO₄:Sm, YVO₄:Dy, LaPO₄:Eu, LaPO₄:Ce, LaPO₄:Ce, Tb, LaPO₄₁Ce,Dy, LaPO₄:Ce, Nd, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, CdS:Mn, ZnS:Tb, ZnS:Ag oder ZnS:Cu als Material für die lad-Nanoteilchen verwendet wird.

17. Einfache Nachweissonde nach einem der Ansprüche 1 bis 16, **dadurch gekennzeichnet, dass** Material mit einer kubischen Gitterstruktur des Wirtsgitters für die lad-Nanoteilchen verwendet wird.

18. Einfache Nachweissonde nach einem der Ansprüche 1 bis 14, **dadurch gekennzeichnet, dass** MgF₂:Mn; ZnS:Mn, ZnS:Ag, ZnS:Cu, CaSiO₃:Ln, CaS:Ln, CaO:Ln, ZnS:Ln, Y₂O₃:Ln, oder MgF₂:Ln (Ln = Lanthaniden) als Material für die lad-Nanoteilchen verwendet wird.

19. Erweiterte Nachweissonde für biologische Anwendungen bestehend aus einer Kombination der einfachen Nachweissonde nach einem der Ansprüche 1 bis 18 mit einem oder mehreren Affinitätsmolekülen oder mehreren miteinander gekoppelten Affinitätsmolekülen, wobei die Affinitätsmoleküle einerseits an die präparierte Oberfläche der einfachen Nachweissonde anbinden und andererseits an eine biologische oder sonstige organische Substanz binden können.

20. Erweiterte Nachweissonde nach Anspruch 19, **dadurch gekennzeichnet, dass** die Affinitätsmoleküle monoklonale oder polyklonale Antikörper, Proteine, Peptide, Oligonukleotide, Plasmide, Nukleinsäuremoleküle, Oligo- oder Polysaccharide, Haptene wie Biotin oder Digoxin, oder ein niedermolekulares synthetisches oder natürliches Antigen sind.

21. Erweiterte Nachweissonde nach Anspruch 19 oder 20, **dadurch gekennzeichnet, dass** das Affinitätsmolekül durch reaktive Gruppen am Affinitätsmolekül und an der einfachen Nachweissonde kovalent oder nicht-kovalent an die einfache Nachweissonde gekoppelt ist.

22. Erweiterte Nachweissonde nach Anspruch 21, **dadurch gekennzeichnet, dass** die reaktiven Gruppen auf der Oberfläche des Affinitätsmoleküls Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen oder Azolide sind.

23. Erweiterte Nachweissonde nach einem der Ansprüche 19 bis 22, **dadurch gekennzeichnet, dass** zwischen der einfachen Nachweissonde und dem Affinitätsmolekül eine nicht-kovalente, selbst-organisierte Verbindung besteht.

24. Erweiterte Nachweissonde nach Anspruch 23, **dadurch gekennzeichnet, dass** eine Verbindung zwischen Biotin als Verbindungsmolekül der einfachen Nachweissonde und Avidin oder Streptavidin als reaktive Gruppe des Affinitätsmoleküls besteht.

25. Erweiterte Nachweissonde nach Anspruch 23, **dadurch gekennzeichnet, dass** eine Verbindung zwischen Nukleinsäuremolekülen als Verbindungsmolekülen der einfachen Nachweissonde und Nukleinsäuremolekülen mit dazu komplementären Sequenzen als reaktiver Gruppe des Affinitätsmoleküls besteht.

26. Erweiterte Nachweissonde nach Anspruch 20, **dadurch gekennzeichnet, dass** als Affinitätsmolekül Nukleinsäuresequenzen dienen und die nachzuweisende biologische Substanz aus Nukleinsäuremolekülen mit komplementären Sequenzen besteht.

27. Verfahren zur Herstellung der einfachen Nachweissonde gemäß einem der Ansprüche 1 bis 18 enthaltend die Schritte
a) Herstellung von lad-Nanoteilchen
b) chemische Modifikation der Oberfläche der lad-Nanoteilchen und/oder
c) Herstellung reaktiver Gruppen auf der Oberfläche der lad-Nanoteilchen und/oder
d) Verbindung eines oder mehrerer Verbindungsmoleküle mit der Oberfläche der lad-Nanoteilchen durch kovalente oder nicht-kovalente Bindung.

28. Verfahren zur Herstellung der einfachen Nachweissonde nach Anspruch 27, **dadurch gekennzeichnet, dass** die Verteilungsbreite der Ausdehnungen der in Schritt a) hergestellten lad-Nanoteilchen auf einen Bereich von +/-20 % einer mittleren Ausdehnung eingeschränkt ist.

29. Verfahren zur Herstellung der erweiterten Nachweissonde gemäß den Ansprüchen 19 bis 26 enthaltend die Schritte
e) Bereitstellung der einfachen Nachweissonde
f) Modifikation der Oberfläche eines Affinitätsmoleküls, um reaktive Gruppen einzuführen, die eine Konjugation mit der einfachen Nachweissonde erlauben
g) Konjugation des aktivierten Affinitätsmoleküls und der einfachen Nachweissonde.

30. Verfahren zum Nachweis einer biologischen oder sonstigen organischen Substanz enthaltend die Schritte
h) Zusammenführen der erweiterten Nachweissonde gemäß einem der Ansprüche 20 bis 27 und des biologischen und/oder organischen Materials,
i) Abtrennung von erweiterten Nachweissonden, die keine Bindung eingegangen sind,
j) Belichtung der Probe mit einer elektromagnetischen Strahlung oder mit einem Teilchenstrahl
k) Messung des Fluoreszenzlichtes oder Messung der Absorption und/oder Streuung und/oder Beugung der Strahlung oder der Änderung derselben.

31. Verfahren nach Anspruch 30, **dadurch gekennzeichnet, dass** es sich bei dem zu untersuchenden biologischen Material um Serum, Zellen, Gewebeschnitte, Rückenmarksflüssigkeit, Sputum, Plasma, Urin oder um eine andere Probe humanen, tierischen oder pflanzlichen Ursprungs handelt.

32. Verfahren nach Anspruch 30 oder 31, **dadurch gekennzeichnet, dass** der zu untersuchende Analyt in dem zu untersuchenden biologischen oder sonstigen Material immobilisiert ist.

33. Verfahren nach einem der Ansprüche 30 bis 32, **dadurch gekennzeichnet, dass** das zu untersuchende biologische und/oder organische Material gleichzeitig mit verschiedenen erweiterten Nachweissonden zusammengeführt wird und die verschiedenen erweiterten Nachweissonden sich **dadurch** voneinander unterscheiden, dass ihre Affinitätsmoleküle an verschiedene Analyte anbinden und die in ihnen enthaltenen lad-Nanoteilchen bei unterschiedlichen Wellenlängen absorbieren, streuen, beugen oder Fluoreszenzlicht emittieren.

## Claims

1. Simple detection probe containing luminescent inorganic doped nanoparticles (lid nanoparticles) which can be detected, after excitation using a radiation source, by absorption and/or scattering and/or diffraction of the exciting radiation or by emission of fluorescent light and whose surface is prepared in such a way that affinity molecules can couple to said prepared surface in order to detect a biological or other organic substance, **characterized in that** the lid nanoparticles have diameters in the range from 2 nm to below 20 nm and **in that** one or more chain-like molecules with a polarity or charge opposite to that of the lid nanoparticle surface are non-covalently linked as linker molecule to the surface of the lid nanoparticles.

2. Simple detection probe according to Claim 1, **characterized in that** the surface of the lid nanoparticles is chemically modified and/or has reactive groups.

3. Simple detection probe according to Claim 1 or 2, **characterized in that** the lid nanoparticles are coated with a layer.

4. Simple detection probe according to Claims 1 to 3, **characterized in that** the surface of the lid particles comprises oxychlorides which are generated by treating lid nanoparticles composed of oxidic transition metal compounds with chlorine gas or organic chlorinating agents.

5. Simple detection probe according to one of Claims 1 to 4, **characterized in that** the chain-like molecules are anionic, cationic or zwitterionic detergents, acidic or basic proteins, polyamines, polyamides or polysulphonic or polycarboxylic acids.

6. Simple detection probe according to any of Claims 1 to 5, **characterized in that** the surface and/or the linker molecules linked to the lid nanoparticle surface carry reactive neutral, charged or partially charged groups such as amino groups, carboxylic acid groups, thiols, thioethers, disulphides, imidazoles, guanidines, hydroxyl groups, indoles, vicinal diols, aldehydes, alpha-haloacetyl groups, N-maleimides, mercury organyls, aryl halides, acid anhydrides, isocyanates, isothiocyanates, sulphonyl halides, imido esters, diazoacetates, diazonium salts, 1,2-diketones, alpha-beta-unsaturated carbonyl compounds, azolides, phosphonic acids, phosphoric esters, or derivatives of said groups, said reactive groups allowing chemical binding to further linker molecules or affinity molecules.

7. Simple detection probe according to any of Claims 1 to 6, **characterized in that** nucleic acid molecules serve as linker molecules for an affinity molecule containing nucleic acid molecules with sequences complementary to said linker molecules.

8. Simple detection probe according to any of Claims 1 to 7, **characterized in that** the radiation source is a source of electromagnetic radiation with wavelengths in the range of infrared light, of visible light, of UV, of X-ray light or of γ radiation or is a source of particle radiation such as electron radiation.

9. Simple detection probe according to any of Claims 1 to 8, **characterized in that** the lid nanoparticles have a needle-like morphology with a width of from 3 nm to 50 nm, preferably from 3 nm to below 20 nm, and a length of from 20 nm to 5 µm, preferably from 20 nm to 500 nm.

10. Simple detection probe according to any of Claims 1 to 9, **characterized in that** the host material of the lid nanoparticles comprises compounds of the XY type, X being a cation of one or more elements of the main groups 1a, 2a, 3a, 4a, of the transition groups 2b, 3b, 4b, 5b, 6b, 7b or of the lanthanides of the Periodic Table and Y being either a polyatomic anion of one or more element(s) of the main groups 3a, 4a, 5a, of the transition groups 3b, 4b, 5b, 6b, 7b and/or 8b and element(s) of the main groups 6a and/or 7 or a monoatomic anion of the main groups 5a, 6a or 7a of the Periodic Table.

11. Simple detection probe according to Claim 10, **characterized in that** the host material of the lid nanoparticles comprises compounds of the group consisting of sulphides, selenides, sulphoselenides, oxysulphides, borates, aluminates, gallates, silicates, germanates, phosphates, halophosphates, oxides, arsenates, vanadates, niobates, tantalates, sulphates, tungstates, molybdates, alkali halides and other halides or nitrides.

12. Simple detection probe according to any of Claims 1 to 11, **characterized in that** one or more elements of the group comprising elements of the main groups 1a, 2a or Al, Cr, Tl, Mn, Ag, Cu, As, Nb, Nd, Ni, Ti, In, Sb, Ga, Si, Pb, Bi, Zn, Co and/or elements of the lanthanides are used as doping agent.

13. Simple detection probe according to Claim 12, **characterized in that** combinations of two or more of said elements at different concentrations relative to one another also serve as doping material.

14. Simple detection probe according to any of Claims 1 to 13, **characterized in that** the concentration of the doping material in the host lattice is between 10⁻⁵ mol% and 50 mol%, preferably between 0.01 mol% and 30 mol%, particularly preferably between 0.1 mol% and 20 mol%.

15. Simple detection probe according to any of Claims 1 to 14, **characterized in that** LiI:Eu; NaI:Tl; CsI:Tl; CsI:Na; LiF:Mg; LiF:Mg,Ti; LiF:Mg,Na; KMgF₃:Mn; Al₂O₃:Eu; BaFCI:Eu; BaFCI:Sm; BaFBr:Eu; BaFCl_{0.5}Br_{0.5}:Sm; BaY₂F₈:A (A = Pr, Tm, Er, Ce); BaSi₂O₅:Pb; BaMg₂Al₁₆O₂₇:Eu; BaMgAl₁₄O₂₃:Eu; BaMgAl₁₀O₁₇:Eu; BaMgAl₂O₃:Eu; Ba₂P₂O₇:Ti; (Ba,Zn,Mg)₃Si₂O₇:Pb; Ce(Mg,Ba)Al₁₁O₁₉; Ce_{0.65}Tb_{0.35}MgAl₁₁O₁₉:Ce,Tb; MgAl₁₁O₁₉:Ce,Tb; MgF₂:Mn; MgS:Eu; MgS:Ce; MgS:Sm; MgS:(Sm,Ce); (Mg,Ca)S:Eu; MgSiO₃:Mn; 3.5MgO·0.5MgF₂·GeO₂:Mn; MgWO₄:Sm; MgWO₄:Pb; 6MgO-As₂O₅:Mn; (Zn,Mg)F₂:Mn; (Zn₄Be)SO₄:Mn; Zn₂SiO₄:Mn; Zn₂SiO₄:Mn,As; ZnO:Zn; ZnO:Zn,Si,Ga; Zn₃(PO₄)₂:Mn; ZnS:A (A = Ag, Al, Cu); (Zn,Cd)S:A (A = Cu, Al, Ag, Ni); CdBO₄:Mn; CaF₂:Mn; CaF₂:Dy; CaS:A A = lanthanides, Bi); (Ca,Sr)S:Bi; CaWO₄:Pb; CaWO₄:Sm; CaSO₄:A (A = Mn, lanthanides); 3Ca₃(PO₄)₂·Ca(F,Cl)₂:Sb,Mₙ; CaSiO₃:Mn,Pb; Ca₂Al₂Si₂O₇:Ce; (Ca,Mg)SiO₃:Ce; (Ca,Mg)SiO3:Ti; 2SrO·6(B₂O₃)·SrF₂:Eu; 3Sr₃(PO₄)₂·CaCl₂:Eu; A₃(PO₄)₂·ACl₂:Eu (A = Sr, Ca, Ba); (Sr,Mg)₂P₂O₇:Eu; (Sr,M₉)₃(PO₄)₂:Sn; SrS:Ce; SrS:Sm,Ce; SrS:Sm; SrS:Eu; SrS:Eu,Sm; SrS:Cu,Ag; Sr₂P₂O₇:Sn; Sr₂P₂O₇:Eu; Sr₄Al₁₄O₂₅:Eu; SrGa₂S₄:A (A = lanthanides, Pb); SrGa₂S₄:Pb; Sr₃Gd₂Si₆O₁₈:Pb,Mn; YF₃:Yb,Er; YF₃:Ln (Ln = lanthanides); YLiF₄:Ln (Ln = lanthanides); Y₃Al₅O₁₂:Ln (Ln = lanthanides); YAl₃(BO₄)₃:Nd,Yb; (Y,Ga)BO₃:Eu; (Y,Gd)BO₃:Eu; Y₂Al₃Ga₂O₁₂:Tb; Y₂SiO₅:Ln (Ln = lanthanides); Y₂O₃:Ln (Ln = lanthanides); Y₂O₂S:Ln (Ln = lanthanides); YVO₄:A (A = lanthanides, In); Y(P,V)O₄:Eu; YTaO₄:Nb; YAlO₃:A (A = Pr, Tm, Er, Ce); YOCI:Yb,Er; LnPO₄:Ce,Tb (Ln = lanthanides or mixtures of lanthanides); LuVO₄:Eu; GdVO₄:Eu; Gd₂O₂S:Tb; GdMgB₅O₁₀:Ce,Th; LaOBr:Tb; La₂O₂S:Tb; LaF₃:Nd,Ce; BaYb₂F₈:Eu; NaYF₄:Yb,Er; NaGdF₄:Yb,Er; NaLaF₄:Yb,Er; LaF₃:Yb,Er,Tm; BaYF₅:Yb,Er; Ga₂O₃:Dy; GaN:A (A = Pr, Eu, Er, Tm); Bi₄Ge₃O₁₂; LiNbO₃:Nd, Yb; LiNbO₃:Er; LiCaAlF₆:Ce; LiSrAlF₆:Ce; LiLuF₄:A (A = Pr, Tm, Er, Ce); Li₂B₄O₇:Mn, SiOₓ:Er, Al (0 ≤ x ≤ 2) is used as material for the lid nanoparticles.

16. Simple detection probe according to any of Claims 1 to 14, **characterized in that** YVO₄:Eu, YVO₄:Sm, YVO₄:Dy, LaPO₄:Eu, LaPO₄:Ce, LaPO₄:Ce,Tb, LaPO₄:Ce,Dy, LaPO₄:Ce,Nd, ZnS:Tb, ZnS:TbF₃, ZnS:Eu, ZnS:EuF₃, Y₂O₃:Eu, Y₂O₂S:Eu, Y₂SiO₅:Eu, SiO₂:Dy, SiO₂:Al, Y₂O₃:Tb, CdS:Mn, ZnS:Tb, ZnS:Ag or ZnS:Cu is used as material for the lid nanoparticles.

17. Simple detection probe according to any of Claims 1 to 16, **characterized in that** material having a cubic host lattice structure is used for the lid nanoparticles.

18. Simple detection probe according to any of Claims 1 to 14, **characterized in that** MgF₂:Mn; ZnS:Mn, ZnS:Ag, ZnS:Cu, CaSiO₃:Ln, CaS:Ln, CaO:Ln, ZnS:Ln, Y₂O₃:Ln, or MgF₂:Ln (Ln = lanthanides) is used as material for the lid nanoparticles.

19. Extended detection probe for biological applications comprising a combination of the simple detection probe according to any of Claims 1 to 18 with one or more affinity molecules or a plurality of affinity molecules coupled to one another, it being possible for said affinity molecules on the one hand to attach to the prepared surface of the simple detection probe and on the other hand to bind to a biological or other organic substance.

20. Extended detection probe according to Claim 19, **characterized in that** the affinity molecules are monoclonal or polyclonal antibodies, proteins, peptides, oligonucleotides, plasmids, nucleic acid molecules, oligo- or polysaccharides, haptens such as biotin or digoxin or a low molecular weight synthetic or natural antigen.

21. Extended detection probe according to Claim 19 or 20, **characterized in that** the affinity molecule is coupled covalently or non-covalently to the simple detection probe via reactive groups on the affinity molecule and on the simple detection probe.

22. Extended detection probe according to Claim 21, **characterized in that** the reactive groups on the affinity molecule surface are amino groups, carboxylic acid groups, thiols, thioethers, disulphides, imidazoles, guanidines, hydroxyl groups, indoles, vicinal diols, aldehydes, alpha-haloacetyl groups, N-maleimides, mercury organyls, aryl halides, acid anhydrides, isocyanates, isothiocyanates, sulphonyl halides, imido esters, diazoacetates, diazonium salts, 1,2-diketones, alpha-beta-unsaturated carbonyl compounds, or azolides.

23. Extended detection probe according to any of Claims 19 to 22, **characterized in that** there is a non-covalent self-organized linkage between the simple detection probe and the affinity molecule.

24. Extended detection probe according to Claim 23, **characterized in that** there is a linkage between biotin as linker molecule of the simple detection probe and avidin or streptavidin as reactive group of the affinity molecule.

25. Extended detection probe according to Claim 23, **characterized in that** there is a linkage between nucleic acid molecules as linker molecules of the simple detection probe and nucleic acid molecules, having sequences complementary thereto, as reactive group of the affinity molecule.

26. Extended detection probe according to Claim 20, **characterized in that** nucleic acid sequences serve as affinity molecule and the biological substance to be detected comprises nucleic acid molecules with complementary sequences.

27. Method for preparing a simple detection probe according to any of Claims 1 to 18, comprising the steps
a) preparation of lid nanoparticles
b) chemical modification of the surface of said lid nanoparticles and/or
c) preparation of reactive groups on the surface of said lid nanoparticles and/or
d) linking one or more linker molecules with the surface of said lid nanoparticles by covalent or non-covalent binding.

28. Method for preparing the simple detection probe according to Claim 27, **characterized in that** the distribution range of the expansions of the lid nanoparticles prepared in step a) is limited to a range of +/- 20% of an average expansion.

29. Method for preparing the extended detection probe according to Claims 19 to 26, comprising the steps
e) providing the simple detection probe
f) modifying the surface of an affinity molecule in order to introduce reactive groups which allow conjugation to the simple detection probe
g) conjugating the activated affinity molecule and the simple detection probe.

30. Method for detecting a biological or other organic substance, comprising the steps
h) combining the extended detection probe according to any of Claims 20 to 27 and the biological and/or organic material,
i) removing extended detection probes which have not bound,
j) exposing the sample to electromagnetic radiation or to a particle beam
k) measuring the fluorescent light or measuring the absorption and/or scattering and/or diffraction of the radiation or the change therein.

31. Method according to Claim 30, **characterized in that** the biological material to be studied is serum, cells, tissue sections, cerebral spinal fluid, sputum, plasma, urine or another sample of human, animal or plant origin.

32. Method according to Claim 30 or 31, **characterized in that** the analyte to be studied is immobilized in the biological or other material to be studied.

33. Method according to any of Claims 30 to 32, **characterized in that** the biological and/or organic material to be studied is combined with different extended detection probes at the same time, and said different extended detection probes differ from one another **in that** their affinity molecules attach to different analytes and the lid nanoparticles contained in said extended detection probes absorb, scatter or diffract or emit fluorescent light at different wavelengths.

## Revendications

1. Sondes individuelles de détection contenant des nanoparticules dopées inorganiques luminescentes (nanoparticules dil), qui après excitation avec une source de rayonnement, sont détectables par absorption et/ou dispersion et/ou diffraction du rayonnement d'excitation ou par émission de lumière fluorescente et dont la surface est préparée de sorte que des molécules d'affinité pour la détection d'une substance biologique ou organique autre peuvent être couplées sur ces surfaces préparées, **caractérisées en ce que** les nanoparticules dil présentent un diamètre situé dans l'intervalle allant de 2 nm à moins de 20 nm et que comme molécule de liaison, une ou plusieurs molécules caténaires avec une polarité ou charge opposée à la surface des nanoparticules dil, sont reliées de manière non covalente à la surface des nanoparticules dil.

2. Sondes individuelles de détection suivant la revendication 1, **caractérisées en ce que** la surface des nanoparticules dil est modifiée chimiquement et/ou présente des radicaux réactifs.

3. Sondes individuelles de détection suivant la revendication 1 ou 2, **caractérisées en ce que** les nanoparticules dil sont enrobées d'une couche de silice.

4. Sondes individuelles de détection suivant l'une des revendications 1 à 3, **caractérisées en ce que** la surface des nanoparticles dil présente des oxychlorures, qui sont formés par le traitement des nanoparticules dil consistant en des composés oxydes de métal de transition avec le chlore gazeux ou des agents organiques de chloration.

5. Sondes individuelles de détection suivant l'une des revendications 1 à 4, **caractérisées en ce que** les molécules caténaires sont des détergents anioniques, cationiques ou zwittérioniques, des protéines acides ou basiques, des polyamines, des polyamides ou des poly(acide sulfoniques ou carboxyliques).

6. Sondes individuelles de détection suivant l'une des revendications 1 à 5, **caractérisées en ce que** la surface et/ou les molécules de liaison reliées à la surface des nanoparticules dil portent des radicaux réactifs neutres, chargés ou partiellement chargés, comme des radicaux amino, des radicaux acide carboxylique, thiol, thioéther, disulfure, imidazole, guanidine, hydroxyle, indole, diols vicinaux, aldéhyde, alpha-haloacétyle, N-maléimide, mercuro-organyle, arylhalogénure, anhydride d'acide, isocyanate, isothiocyanate, halogénure d'acide sulfonique, imidoester, diazoacétate, sel de diazonium, 1,2-dicétone, composé carbonyle alpha,bêta-insaturé, azolide, acide phosphonique, ester d'acide phosphorique ou des dérivés des radicaux indiqués, où ces radicaux réactifs permettent une liaison chimique avec d'autres molécules de liaison ou molécules d'affinité.

7. Sondes individuelles de détection suivant l'une des revendications 1 à 6, **caractérisées en ce que** des molécules d'acide nucléique servent de molécules de liaison pour une molécule d'affinité contenant une molécule d'acide nucléique avec séquence complémentaire à la molécule de liaison.

8. Sondes individuelles de détection suivant l'une des revendications 1 à 7, **caractérisées en ce que** la source de rayonnement est une source de rayonnement électromagnétique de longueurs d'onde situées dans l'intervalle de la lumière infra-rouge, de la lumière visible, des U.V., des rayons X ou du rayonnement γ ou est une source de rayonnement particulaire comme un rayonnement électronique.

9. Sondes individuelles de détection suivant l'une des revendications 1 à 8, **caractérisées en ce que** les nanoparticles dil présentent une morphologie en forme d'aiguilles, avec une largeur allant de 3 nm à 50 nm, de préférence de 3 nm à moins de 20 nm et une longueur allant de 20 nm à 5 µm, de préférence de 20 nm à 500 nm.

10. Sondes individuelles de détection suivant l'une des revendications 1 à 9, **caractérisées en ce que** le matériau hôte des nanoparticules dil contient des composés de type XY, où X est un cation d'un ou de plusieurs éléments des groupes principaux 1a, 2a, 3a, 4a, des groupes secondaires 2b, 3b, 4b, 5b, 6b, 7b ou des lanthanides du système périodique et Y est un anion polyatomique d'un ou de plusieurs éléments des groupes principaux 3a, 4a, 5a, des groupes secondaires 3b, 4b, 5b, 6b, 7b et/ou 8b ainsi que des éléments des groupes principaux 6a et/ou 7a ou un anion monoatomique des groupes principaux 5a, 6a ou 7a du système périodique.

11. Sondes individuelles de détection suivant la revendication 10, **caractérisées en ce que** le matériau hôte des nanoparticules dil contient des composés du groupe des sulfures, sélénures, sulfosélénures, oxysulfures, borates, aluminates, gallates, silicates, germanates, phosphates, halophosphates, oxydes, arséniates, vanadates, niobates, tantalates, sulfates, tungstates, molybdates, halogénures alcalins ainsi que d'autres halogénures ou nitrures.

12. Sondes individuelles de détection suivant l'une des revendications 1 à 11, **caractérisées en ce que** comme dopage, on utilise un ou plusieurs éléments du groupe contenant les éléments les groupes principaux 1a, 2a ou Al, Cr, Tl, Mn, Ag, Cu, As, Nb, Nd, Ni, Ti, In, Sb, Ga, Si, Pb, Bi, Zn, Co et/ou des éléments des lanthanides.

13. Sondes individuelles de détection suivant la revendication 12, **caractérisées en ce que** des combinaisons de deux ou plusieurs de ces éléments en différentes concentrations relatives l'une par rapport à l'autre servent comme matériau de dopage.

14. Sondes individuelles de détection suivant l'une des revendications 1 à 13, **caractérisées en ce que** la concentration du matériau de dopage dans le réseau hôte se situe dans l'intervalle allant de 10⁻⁵% en moles à 50% en moles, de préférence de 0,01% en moles à 30% en moles, de manière particulièrement préférée, de 0,1% en moles à 20% en moles.

15. Sondes individuelles de détection suivant l'une des revendications 1 à 14, **caractérisées en ce que** l'on utilise comme matériau pour les nanoparticules dil, LiI:Eu ; NaI:Tl ; CsI:Tl ; CsI:Na ; LiF:Mg ; LiF:Mg,Ti ; LiF:Mg,Na ; KMgF₃:Mn, Al₂O₃:Eu; BaFCl :Eu ; BaFCl:Sm; BaFBr:Eu; BaFCl_{0,5}Br_{0,5}:Sm; BaY₂F₈:A (A = Pr, Tm, Er, Ce); BaSi₂O₅:Pb; BaMg₂Al₁₆O₂₇:Eu; BaMgAl₁₄O₂₃:Eu; BaMgAl₁₀O₁₇:Eu; BaMgAl₂O₃:Eu; Ba₂P₂O₇:Ti; (Ba,Zn,Mg)₃Si₂O₇:Pb; Ce (Mg, Ba) Al₁₁O₁₉; Ce_{0,65}Tb_{0,35}MgAl₁₁O₁₉:Ce, Tb; MgAl₁₁O₁₉:Ce, Tb; MgF₂:Mn; MgS:Eu ; MgS:Ce; MgS:Sm ; MgS:(Sm,Ce); (Mg,Ca)S:Eu; MgSiO₃:Mn; 3, 5MgO. 0,5MgF2.GeO₂:Mn; MgWO₄:Sm; MgWO₄:Pb; 6MgO.As₂O₅:Mn; (Zn,Mg)F₂:Mn; (Zn₄Be)SO₄:Mn; Zn₂SiO₄:Mn; Zn₂SiO₄:Mn, As; ZnO: Zn; ZnO:Zn, Si, Ga; Zn₃(PO₄)₂:Mn ; ZnS:A (A = Ag, Al, Cu) ; (Zn,Cd)S:A (A = Cu, Al, Ag, Ni) ; CdBO₄:Mn ; CaF₂:Mn ; CaF₂:Dy ; CaS:A (A = lanthanide, Bi) ; (Ca, Sr) S:Bi ; CaWO₄:Pb ; CaWO₄:Sm ; CaSO₄:A (A = Mn, lanthanide) ; 3Ca₃(PO₄)₂.Ca(F,Cl)₂:Sb,Mn ; CaSiO₃:Mn,Pb ; Ca₂Al₂Si₂O₇:Ce ; (Ca,Mg)SiO₃:Ce ; (Ca,Mg)SiO₃:Ti ; 2SrO.6 (B₂O₃).SrF₂:Eu ; 3Sr₃(PO₄)₂.CaCl₂:Eu ; A₃(PO₄)₂.ACl₂:Eu (A = Sr, Ca, Ba) ; (Sr,Mg)₂P₂O₇:Eu ; (Sr, Mg)₃(PO₄)₂:Sn ; SrS:Ce ; SrS:Sm, Ce ; SrS:Sm ; SrS:Eu ; SrS:Eu,Sm ; SrS:Cu, Ag ; Sr₂P₂O₇:Sn ; Sr₂P₂O₇:Eu ; Sr₄Al₁₄O₂₅: Eu; SrGa₂S₄:A (A = lanthanide, Pb) ; SrGa₂S₄:Pb ; Sr₃Gd₂Si₆O₁₈:Pb,Mn ; YF₃:Yb,Er ; YF₃:Ln (Ln = lanthanide) ; YLiF₄:Ln (Ln = lanthanide) ; Y₃Al₅O₁₂:Ln (Ln = lanthanide) ; YAl₃(BO₄)₃:Nd,Yb ; (Y,Ga)BO₃:Eu ; (Y,Gd)BO₃:Eu ; Y₂Al₃Ga₂O₁₂:Tb ; Y₂SiO₅:Ln (Ln = lanthanide) ; Y₂O₃:Ln (Ln = lanthanide) ; Y₂O₂S:Ln (Ln = lanthanide) ; YVO₄:A (A = lanthanide, In) ; Y(P,V)O₄:Eu ; YTaO₄:Nb ; YAlO₃:A (A = Pr, Tm, Er, Ce) ; YOCl:Yb,Er ; LnPO₄:Ce,Tb (Ln = lanthanide ou des mélanges de lanthanides) ; LuVO₄:Eu ; GdVO₄:Eu ; Gd₂O₂S:Tb : GdMgB₅O₁₀:Ce,Tb ; LaOBr:Tb ; La₂O₂S:Tb ; LaF₃:Nd,Ce; BaYb₂F₈:Eu ; NaYF₄:Yb,Eu ; NaGdF₄:Yb,Er ; NaLaF₄:Yb,Er ; LaF₃:Yb,Er,Tm ; BaYF₅:Yb,Er ; Ga₂O₃:Dy ; GaN:A (A = Pr, Eu, Er, Tm) ; Bi₄Ge₃O₁₂ ; LiNbO₃:Nd,Yb ; LiNbO₃:Er ; LiCaAlF₆:Ce ; LiSrAlF₆:Ce ; LiLuF₄:A (A = Pr, Tm, Er, Ce) ; Li₂B₄O₇:Mn; SiOₓ:Er, Al (0 ≤ x ≤ 2).

16. Sondes individuelles de détection suivant l'une des revendications 1 à 14, **caractérisées en ce que** l'on utilise comme matériau pour les nanoparticules dil, YVO₄:Eu, YVO₄:Sm, YVO₄:Dy, LaPO₄:Eu, LaPO₄:Ce, LaPO₄:Ce,Tb, LaPO₄:Ce,Dy, LaPO₄:Ce,Nd, ZnS:Tb, ZnS:TbF₃, ZnS:Eu, ZnS:EuF₃, Y₂O₃:Eu, Y₂O₂S:Eu, Y₂SiO₅:Eu ; SiO₂:Dy ; SiO₂:Al, Y₂O₃:Tb, CdS : Mn, ZnS : Tb, ZnS:Ag ou ZnS:Cu.

17. Sondes individuelles de détection suivant l'une des revendications 1 à 16, **caractérisées en ce qu'**un matériau avec une structure cristalline cubique du réseau hôte est utilisé comme nanoparticules dil.

18. Sondes individuelles de détection suivant l'une des revendications 1 à 14, **caractérisées en ce que** l'on utilise comme matériau pour les nanoparticules dil, MgF₂:Mn, ZnS:Mn, ZnS:Ag, ZnS:Cu, CaSiO₃:Ln, CaS : Ln, CaO : Ln, ZnS : Ln, Y₂O₃:Ln ou MgF₂:Ln (Ln = lanthanide).

19. Sondes de détection élargie pour utilisations biologiques, consistant en une combinaison des sondes individuelles de détection suivant l'une des revendications 1 à 18, avec une ou plusieurs molécules d'affinité ou plusieurs molécules d'affinité couplées les unes avec les autres, où les molécules d'affinité se lient à la surface préparée des sondes de détection individuelles et peuvent lier d'autre part, une substance biologique ou organique autre.

20. Sondes de détection élargie suivant la revendication 19, **caractérisées en ce que** les molécules d'affinité sont des anticorps monoclonaux ou polyclonaux, des protéines, des peptides, des oligonucléotides, des plasmides, des molécules d'acide nucléique, des oligo- ou polysaccharides, des haptènes comme la biotine ou la digoxine, ou un antigène synthétique ou naturel, de faible poids moléculaire.

21. Sondes de détection élargie suivant la revendication 19 ou 20, **caractérisées en ce que** les molécules d'affinité sont couplées de manière covalente ou non covalente sur la sonde de détection individuelle, par des radicaux réactifs sur la molécule d'affinité et sur la sonde individuelle de détection.

22. Sondes de détection élargie suivant la revendication 21, **caractérisées en ce que** les radicaux réactifs sur la surface des molécules d'affinité sont des radicaux amino, des radicaux acide carboxylique, thiol, thioéther, disulfure, imidazole, guanidine, hydroxyle, indole, diols vicinaux, aldéhyde, alpha-haloacétyle, N-maléimide, mercuro-organyle, arylhalogénure, anhydride d'acide, isocyanate, isothiocyanate, halogénure d'acide sulfonique, imidoester, diazoacétate, sel de diazonium, 1,2-dicétone, composé carbonyle alpha,bêta-insaturé ou azolide.

23. Sondes de détection élargie suivant l'une des revendications 19 à 22, **caractérisées en ce qu'**entre la sonde individuelle de détection et la molécule d'affinité, il y a une liaison non covalente, auto-organisée.

24. Sondes de détection élargie suivant la revendication 23, **caractérisées en ce qu'**il y a une liaison entre la biotine comme molécule de liaison de la sonde individuelle de détection et l'avidine ou la streptavidine comme radical réactif de la molécule d'affinité.

25. Sondes de détection élargie suivant la revendication 23, **caractérisées en ce qu'**il y a une liaison entre des molécules d'acide nucléique comme molécule de liaison de la sonde de détection individuelle et des molécules d'acide nucléique avec des séquences complémentaires comme radical réactif de la molécule d'affinité.

26. Sondes de détection élargie suivant la revendication 20, **caractérisées en ce que** comme molécule d'affinité, on utilise des séquences d'acide nucléique et la substance biologique à détecter consiste en des molécules d'acide nucléique de séquence complémentaire.

27. Procédé de préparation des sondes individuelles de détection suivant l'une des revendications 1 à 18, comprenant les étapes de :
a) préparation de nanoparticules dil ;
b) modification chimique de la surface des nanoparticules dil, et/ou
c) préparation des radicaux réactifs sur la surface des nanoparticules dil, et/ou
d) liaison d'une ou de plusieurs molécules de liaison à la surface des nanoparticules dil, par liaison covalente ou non covalente.

28. Procédé de préparation de sondes individuelles de détection suivant la revendication 27, **caractérisé en ce que** la largeur de distribution de la dimension des nanoparticules préparées à l'étape a) est limitée dans un intervalle de +/- 20% de la dimension moyenne.

29. Procédé de préparation des sondes de détection élargie suivant l'une des revendications 19 à 26, comprenant les étapes de :
e) préparation de sondes individuelles de détection ;
f) modification de la surface d'une molécule d'affinité, pour introduire des radicaux réactifs, qui permettent une conjugaison avec les sondes individuelles de détection ;
g) conjugaison des molécules d'affinité activées et des sondes individuelles de détection.

30. Procédé de détection d'une substance biologique ou organique autre, contenant les étapes de :
h) réunion des sondes de détection élargie suivant l'une des revendications 20 à 27 et du matériau biologique et/ou organique ;
i) séparation des sondes de détection élargie, qui ne sont pas engagées dans une liaison ;
j) illumination de l'échantillon avec un rayonnement électromagnétique ou avec un rayon particulaire ;
k) mesure de la lumière fluorescente ou mesure de l'absorption et/ou de la dispersion et/ou de la diffraction du rayonnement ou de sa modification.

31. Procédé suivant la revendication 30, **caractérisé en ce que** le matériau biologique à étudier consiste en le sérum, des cellules, une coupe tissulaire, le fluide de moelle épinière, le fluide d'expectoration, le plasma, l'urine ou en un autre échantillon d'origine humaine, animale ou végétale.

32. Procédé suivant la revendication 30 ou 31, **caractérisé en ce que** l'analyte à étudier est immobilisé dans le matériau biologique ou autre à étudier.

33. Procédé suivant l'une des revendications 30 à 32, **caractérisé en ce que** le matériau biologique et/ou organique à étudier est réuni avec différentes sondes de détection élargie et les différentes sondes de détection élargie se différencient les unes des autres **en ce que** leurs molécules d'affinité se lient à différentes analytes et que les nanoparticules dil qu'elles contiennent, absorbent, dispersent, diffractent ou émettent de la lumière fluorescente à différentes longueurs d'onde.
